# EUROPEAN PATENT SPECIFICATION

(11) **EP 4 563 084 B1**
(45) Date of publication and mention of the grant of the patent: **18.11.2026**
(21) Application number: 23859975.7
(22) Date of filing: 04.08.2023
(51) Int. Cl.: A61B 5/107, G16H 40/63, G16H 50/30

(54) **EVALUATION DEVICE, EVALUATION METHOD, AND EVALUATION PROGRAM**
AUSWERTUNGSVORRICHTUNG, AUSWERTUNGSVERFAHREN UND AUSWERTUNGSPROGRAMM
DISPOSITIF D'ÉVALUATION, PROCÉDÉ D'ÉVALUATION, ET PROGRAMME D'ÉVALUATION

(30) Priority: 29.08.2022 JP 2022136115
(43) Date of publication of application: 04.06.2025
(73) Proprietor: ASICS Corporation, Kobe-shi, Hyogo 650-0021 (JP)
(72) Inventor: ICHIKAWA, Masaru, Kobe-shi, Hyogo 650-0021 (JP); KUSUMI, Hiroyuki, Kobe-shi, Hyogo 650-0021 (JP); KUSANO, Ken, Kobe-shi, Hyogo 650-0021 (JP); KIKUCHI, Kyota, Tokyo 155-0031 (JP); KIKUCHI, Mamoru, Tokyo 155-0031 (JP)
(74) Representative: TBK
(86) International application number: PCT/JP2023/028614
(87) International publication number: WO 2024/048207

(56) References cited:
- EP-A1- 1 618 806
- CN-A- 104 366 906
- CN-A- 111 713 806
- CN-A- 112 353 383
- JP-A- 2004 305 374
- KR-A- 20200 121 550
- KR-A- 20210 046 944
- YAMASHITA TOMOKO ET AL: "Analysis of skeletal characteristics of flat feet using three-dimensional foot scanner and digital footprint", BIOMEDICAL ENGINEERING ONLINE, vol. 21, no. 1, 15 August 2019 (2019-08-15), GB, pages 56, XP093144906, ISSN: 1475-925X, DOI: 10.1186/s12938-022-01021-7
- YAMASHITA TOMOKO, YAMASHITA KAZUHIKO, SATO MITSURU, KAWASUMI MASASHI, ATA SHINGO: "Analysis of skeletal characteristics of flat feet using three-dimensional foot scanner and digital footprint", BIOMEDICAL ENGINEERING ONLINE, BIOMED CENTRAL LTD, LONDON, GB, vol. 21, no. 1, 15 August 2019 (2019-08-15), GB , pages 56, XP093144906, ISSN: 1475-925X, DOI: 10.1186/s12938-022-01021-7
- ARUNTAMMANAK WANLOP; AUNHATHAWEESUP YUTTAPONG; WONGSEREE WARANYU; LEELASANTITHAM ADISORN; KIATTISIN SUPAPORN: "Diagnose flat foot from foot print image based on neural network", THE 6TH 2013 BIOMEDICAL ENGINEERING INTERNATIONAL CONFERENCE, IEEE, 23 October 2013 (2013-10-23), pages 1 - 5, XP032531665, DOI: 10.1109/BMEiCon.2013.6687684
- YAMASHITA KAZUHIKO; YAMASHITA TOMOKO; SATO MITSURU; KAWASUMI MASASHI; TAKASE YOSHIMASA: "Development of a quantitative measurement system for three-dimensional analysis of foot morphology using a smartphone*", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 3171 - 3174, XP033625117, DOI: 10.1109/EMBC.2019.8857209

## Description

### TECHNICAL FIELD

The present disclosure relates to an evaluation device, an evaluation method, and an evaluation program.

### BACKGROUND

Human feet have shapes including several characteristic features, one of which is a flat foot. For example, NPL 1 discloses a technique for evaluating whether a subject's foot is a flat foot or not by irradiating the foot with X-rays.

### CITATION LIST

### PATENT LITERATURE

PL 1: CN 112 353 383 A "Foot Shape Detection Method and Foot Shape Scanner"
PL 2: EP 1 618 806 A1 "Foot Tilt Angle Measuring Method, Method of Selecting Shoe or Insole for Shoe, Method of Manufacturing Shoe or Insole for Shoe, and Foot Tilt Angle Measuring Device"

### NON PATENT LITERATURE

NPL 1: The Japanese Orthopaedic Association, "Screening Symptoms/Diseases", [online], [searched on August 4, 2022], Internet <URL: https://www.joa.or.jp/public/sick/condition/adult_period_flatfoot.html>
NPL 2: Yamashita Tomoko ET AL: "Analysis of skeletal characteristics of flat feet using three-dimensional foot scanner and digital footprint", BIOMEDICAL ENGINEERING ONLINE, vol. 21, no. 1, 15 August 2019 (2019-08-15), page 56, XP093144906, GB ISSN: 1475-925X, DOI: 10.1186/s12938-022-01021-7

### SUMMARY

### TECHNICAL PROBLEM

According to the above-described technique, the subject's foot needs to be irradiated with X-rays. This may cause a problem that a burden such as X-ray irradiation on the subject increases.

The present disclosure has been made to solve the above-described problems, and an object of an aspect is to evaluate a flat-foot level of a foot of a subject while suppressing a burden on the subject.

### SOLUTION TO PROBLEM

An evaluation device of the present disclosure is defined in claims 1 to 21.

An evaluation method of the present disclosure is defined in claim 22.

### ADVANTAGEOUS EFFECTS OF INVENTION

According to the present disclosure, the flat-foot level of the foot of the subject can be evaluated while suppressing a burden on the subject.

### BRIEF DESCRIPTION OF THE DRAWINGS

Fig. 1 is a diagram for illustrating a configuration of an evaluation system according to the present embodiment.
Fig. 2 is a diagram for illustrating an example of a hardware configuration of an evaluation device.
Fig. 3 is a diagram for illustrating eight index values in the present embodiment.
Fig. 4 is a diagram for illustrating index values related to a frontal plane.
Fig. 5 is a diagram for illustrating index values related to a sagittal plane.
Fig. 6 is a diagram for illustrating index values related to a horizontal plane.
Fig. 7 is a diagram for illustrating a cross section of a foot.
Fig. 8 is a diagram for illustrating an arch line.
Fig. 9 is a functional block diagram of the evaluation device.
Fig. 10 is a diagram for illustrating a correlation between the index values in the present embodiment and X-ray index values.
Fig. 11 is a diagram for illustrating an example showing correlation coefficients between the eight index values in the present embodiment and seven X-ray index values.
Fig. 12 is a diagram for illustrating threshold values and the like in the present embodiment.
Fig. 13 is a flowchart illustrating a main process of the evaluation device.
Fig. 14 is a diagram for illustrating a table used by the evaluation device.
Fig. 15 is a diagram for illustrating a first prescribed range.
Fig. 16 is a diagram for illustrating a second prescribed range.
Fig. 17 is a diagram for illustrating a third prescribed range.
Fig. 18 is a diagram for illustrating an example showing calculated scores and the like.
Fig. 19 is a diagram for illustrating an example of a display screen of a display 3.
Fig. 20 is a diagram for illustrating an example of another flat-foot type image.
Fig. 21 is a diagram showing an example of a third coordinate image.
Fig. 22 is an example showing correspondences between attributes and ranges corresponding to the attributes.

### DETAILED DESCRIPTION

Hereinafter, embodiments will be described with reference to the accompanying drawings. In the following description, the same components are denoted by the same reference characters. Their names and functions are also the same. Therefore, the detailed description thereof will not be repeated.

### <First Embodiment>

### [Configuration of Evaluation System]

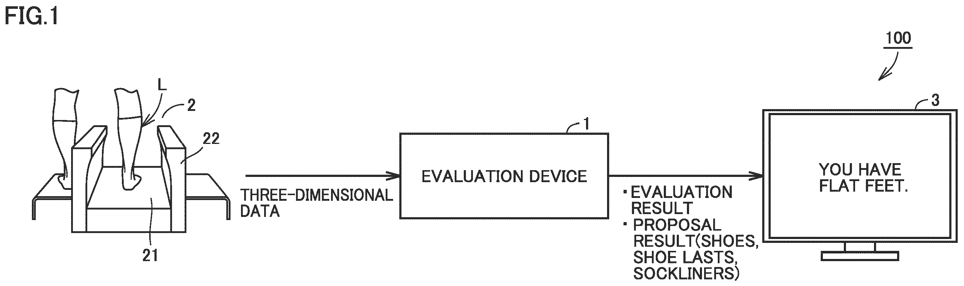

Fig. 1 is a schematic diagram showing a configuration of an evaluation system 100 according to the first embodiment. In the example in Fig. 1, the evaluation system 100 includes an evaluation device 1, a measurement device 2, and a display 3. The measurement device 2 is, for example, a three-dimensional foot shape scanner with laser measurement, and includes a top plate 21 and a laser measurement unit 22 disposed to sandwich the top plate.

A subject places his/her foot L on the top plate 21. While the laser measurement unit 22 moves from a toe to a heel of the foot L, the measurement device 2 measures the shape of the foot of the subject who is standing still. The measurement device 2 outputs the three-dimensional data of the shape of the subject's foot that has been acquired by the laser measurement unit 22 to the evaluation device 1.

The evaluation device 1 evaluates the flat-foot level of the subject's foot based on eight index values, which will be described later. The flat-foot level is, for example, information indicating the flat foot degree. In the first embodiment, the flat-foot level is information indicating whether or not the foot is a flat foot. Then, the evaluation device 1 generates an evaluation result regarding the flat-foot level. In the first embodiment, the evaluation result is information indicating whether the subject's foot belongs to a flat foot group or a non-flat foot group. The "flat foot" is characterized in that an arch of the sole of the foot L collapses, so that the foot sole becomes flat. The "groups" are classified based on the shape of the arch of the foot of each subject. Further, the evaluation device 1 may generate a proposal result based on the eight index values. The "proposal result" is information indicating at least one of: a shoe to be fitted to the subject's foot L; a shoe last to be fitted to the subject's foot L; a sockliner of the shoe to be fitted to the subject's foot L; foot care suitable for the subject's foot L; and training (heel raise and the like) suitable for the subject.

The evaluation device 1 outputs the evaluation result and the proposal result to the display 3. The display 3 shows an image formed based on the evaluation result and the proposal result. For example, when the evaluation result indicates, "the subject's feet L are flat feet", the display 3 shows an image indicating that the foot L is a flat foot. Fig. 1 shows an example in which the display 3 shows a character image stating, "you have flat feet".

On the other hand, when the evaluation result indicates "the subject's feet L are not flat feet", the display 3 shows an image indicating that the foot L is not a flat foot.

### [Hardware Configuration of Evaluation Device]

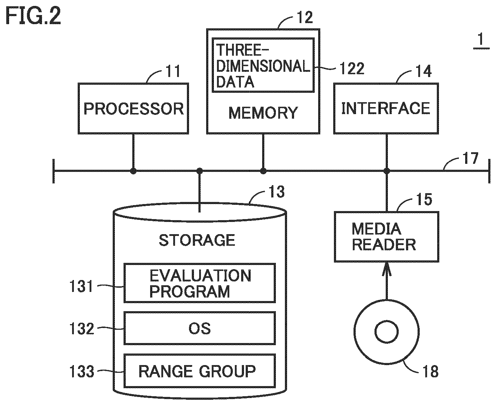

Fig. 2 is a diagram showing an example of a hardware configuration of the evaluation device 1. As shown in Fig. 2, the evaluation device 1 includes a processor 11, a memory 12, a storage 13, an interface 14, and a media reader 15. These components are connected to each other via a processor bus 17.

The storage 13 is implemented, for example, by a nonvolatile storage device such as a hard disk drive (HDD) or a solid state drive (SSD). The storage 13 stores an evaluation program 131, an operating system (OS) 132, and a range group 133.

The evaluation program 131 is a program for executing a process of evaluating the flat-foot level of the subject's foot L based on eight index values, which will be described later. The range group 133 includes a first range to an eighth range (described later) and an overall threshold value.

The processor 11 is a computer that reads the evaluation program 131 and the OS (operating system) 132 stored in the storage 13, deploys the read program onto the memory 12, and executes the program. The processor 11 is implemented, for example, by a central processing unit (CPU), a field programmable gate array (FPGA), a graphics processing unit (GPU), a multi-processing unit (MPU), or the like. The processor 11 may be implanted by processing circuitry.

The memory 12 is implemented by a volatile memory such as a dynamic random access Memory (DRAM) or a static random access memory (SRAM), a nonvolatile memory such as a read only memory (ROM) or a flash memory, or the like. As will be described later, the memory 12 temporarily stores the three-dimensional data transmitted from the measurement device 2.

The interface 14 transmits and receives data to and from other devices through wired or wireless communication. For example, the evaluation device 1 receives (acquires) three-dimensional data from the measurement device 2 via the interface 14. Further, the evaluation device 1 transmits the evaluation result to the display 3 via the interface 14.

The media reader 15 receives a recording medium such as a removable disk 18, and acquires data stored in the removable disk 18.

Further, the evaluation program 131 may be provided as a program product that can be downloaded by an information provider through the Internet. In this case, the processor 11 reads the evaluation program 131 through the Internet.

Further, the evaluation program 131 stored in a recording medium may be distributed. This recording medium is a non-transitory medium from which a computer can read the evaluation program 131 stored in the recording medium. The recording medium is, for example, a digital versatile disk read only memory (DVD-ROM), a compact disc read-only memory (CD-ROM), and the like.

### [Eight Index Values]

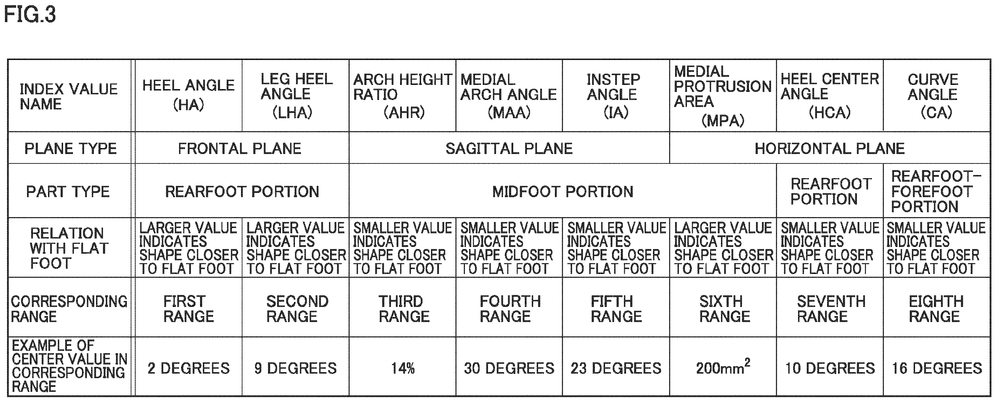

Fig. 3 is a diagram showing eight index values in the present embodiment. The evaluation device 1 evaluates the flat-foot level based on these eight index values. These eight index values specify the shape of a foot. Regarding these eight index values, the example in Fig. 3 shows an index value name, a plane type of the foot L, a part type of the foot L, a relation with a flat foot, a corresponding range, and a specific example of a center value in the corresponding range.

As indicated in the row of the index value name, the eight index values include: a heel angle (HA); a leg heel angle (LHA); an arch height ratio (AHR); a medial arch angle (MAA); an instep angle (IA); a medial protrusion area (MPA); a heel center angle (HCA); and a curve angle (CA).

Hereinafter, the heel angle is also referred to as "HA". The leg heel angle is also referred to as "LHA". The arch height ratio is also referred to as "AHR". The medial arch angle is also referred to as "MAA".

Further, the instep angle is also referred to as "IA". The medial protrusion area is also referred to as "MPA". The heel center angle is also referred to as "HCA". The curve angle is also referred to as "CA".

Further, as indicated in the row of the "plane type", the HA and the LHA each are an "index value related to a frontal plane of a foot". The AHR, the MAA, and the IA each are an "index value related to a sagittal plane of a foot". The MPA, the HCA, and the CA each are an "index value related to a horizontal plane of a foot".

Further, as indicated in the row of the "part type", the HA, the LHA, and the HCA each are an index value related to a rearfoot portion of a foot. The AHR, the MAA, the IA, and the MPA each are an index value related to a midfoot portion of a foot. The CA is an index value related to a forefoot portion and a rearfoot portion of a foot.

The physical quantities of the HA, the LHA, the MAA, the IA, the HCA, and the CA are angles and the physical quantity of the MPA is a "square millimeter". The AHR is expressed in percentage (%).

Further, as indicated in the row of the "relation with flat foot", the HA, the LHA, and the MPA each are an index value having a tendency that a larger value indicates a shape closer to a flat foot. Further, the AHR, the MAA, the IA, the HCA, and the CA each are an index value having a tendency that a smaller value indicates a shape closer to a flat foot.

Further, as indicated also in the row of the corresponding range, the HA, the LHA, the AHR, the MAA, the IA, the MPA, the HCA, and the CA are compared with the first range, the second range, the third range, the fourth range, the fifth range, the sixth range, the seventh range, and the eighth range, respectively. This comparison will be described later.

The center value of the first range is 2 degrees, and the center value of the second range is 9 degrees. The center value of the third range is 14%, and the center value of the fourth range is 30 degrees. The center value of the fifth range is 23 degrees, and the center value of the sixth range is 200 square millimeters. The center value of the seventh range is 10 degrees, and the center value of the eighth range is 16 degrees.

Further, the upper limit value of each range is defined by adding a prescribed value to the center value of the corresponding range, and the lower limit value of each range is defined by subtracting the prescribed value from the center value of the corresponding range. For example, the first range is 1 degree or more and 3 degrees or less when the prescribed value is set as 1 degree.

The center value of the first range may be any value from 0 degrees to 5 degrees. The center value of the second range may be any value from 5 degrees to 10 degrees. The center value of the third range may be any value from 10% to 15%. The center value of the fourth range may be any value from 25 degrees to 30 degrees. The center value of the fifth range may be any value from 20 degrees to 25 degrees. The center value of the sixth range may be any value from 195 square millimeters to 205 square millimeters. The center value of the seventh range may be any value from 5 degrees to 10 degrees. The center value of the eighth range may be any value from 15 degrees to 20 degrees. Further, the prescribed value of each range is set by a measuring person or the like. Note that "A to B" indicates, for example, a range of A or more and B or less.

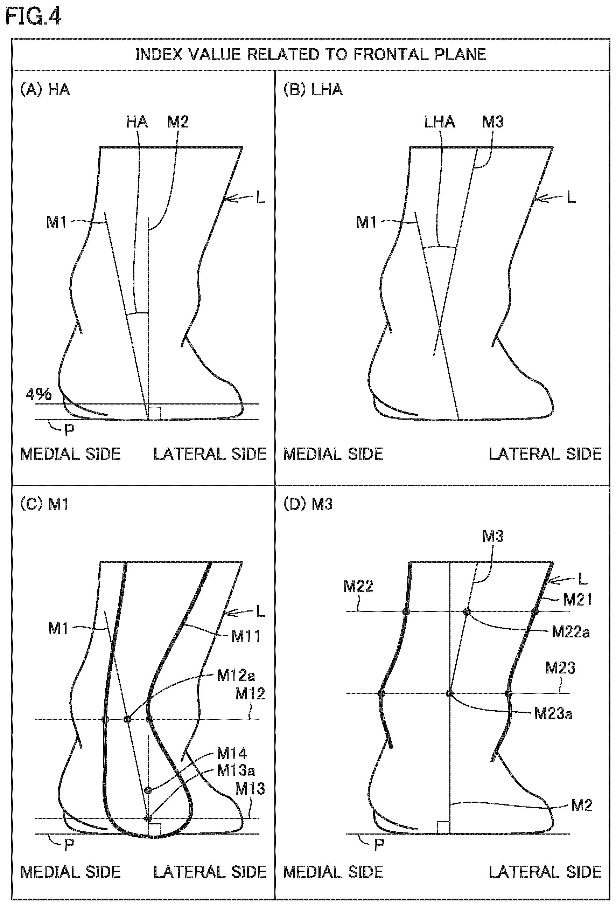

Next, each index value will be described in detail. Fig. 4 is a diagram for illustrating the HA and the LHA, each of which is an index value related to a frontal plane. In Fig. 4, a heel portion of the right foot of the subject is shown as the foot L. Further, Fig. 4 shows a medial side and a lateral side of the right foot (the foot L).

Fig. 4(A) is a diagram for illustrating the HA. Fig. 4(A) shows a heel midline M1 and a vertical axis M2 of the foot. The heel midline M1 will be described later with reference to Fig. 4(C). The vertical axis M2 is an axis orthogonal to a ground contact surface P of the foot L. The HA is an angle formed between the heel midline M1 and the vertical axis M2.

Fig. 4(B) is a diagram for illustrating the LHA. Fig. 4(B) shows the heel midline M1 and a leg axis M3. The leg axis M3 will be described later with reference to Fig. 4(D). The LHA is an angle formed between the heel midline M1 and the leg axis M3. Further, as described with reference to Fig. 3, the HA and the LHA each are an index value having a tendency that a larger value is determined to indicate a shape closer to a flat foot.

Further, in the present embodiment, the expression "A% of the foot length (A is a real number)" indicates the "position of the length at A% of the foot length from the heel portion of the foot L". Further, A% includes approximately A%. The "foot length" is, for example, the length between the heel point and the point where the tip end of the longest toe falls on the center line of the foot. Further, A% may be a value different from the value disclosed in the present embodiment, for example, within a range in which the evaluation device 1 can make an appropriate evaluation about a flat foot.

Fig. 4(C) is a diagram for illustrating the heel midline M1. Fig. 4(C) shows a curved line M11. The curved line M11 is a contour line of a prescribed cross section of the foot on the frontal plane at any position of 0% to 10% of the foot length. Further, Fig. 4(C) shows straight lines M12 and M13. The straight line M12 is a straight line parallel to the ground contact surface P and located on the above-mentioned prescribed cross section at a position at any length of 20% to 30% of the foot length from the ground contact surface P of the foot L. The straight line M13 is a straight line parallel to the ground contact surface P and located on the above-mentioned prescribed cross section at a position at any length of 1% to 10% of the foot length from the ground contact surface P of the foot L.

A midpoint between two points at which the straight line M12 intersects with the curved line M11 is defined as a midpoint M12a. Further, a point at which a perpendicular line passing through a heel point M14 among perpendicular lines of the straight line M13 intersects with the straight line M13 is defined as an intersection point M13a. The heel midline M1 is a straight line connecting the midpoint M12a and the intersection point M13a.

Fig. 4(D) is a diagram for illustrating the leg axis M3. Fig. 4(D) shows a curved line M21. The curved line M21 is a contour line of the ankle. Further, Fig. 4(D) shows straight lines M22 and M23. The straight line M22 is a straight line parallel to the ground contact surface P and located at a position at any length of 50% to 60% of the foot length from the ground contact surface P of the foot L. The straight line M23 is a straight line parallel to the ground contact surface P and located at a position at any length of 30% to 40% of the foot length from the ground contact surface P of the foot L.

Further, a midpoint between two points at which the straight line M22 intersects with the curved line M21 is defined as a midpoint M22a. A midpoint between two points at which the straight line M23 intersects with the curved line M21 is defined as a midpoint M23a. The leg axis M3 is a straight line connecting the midpoint M12a and the intersection point M13a.

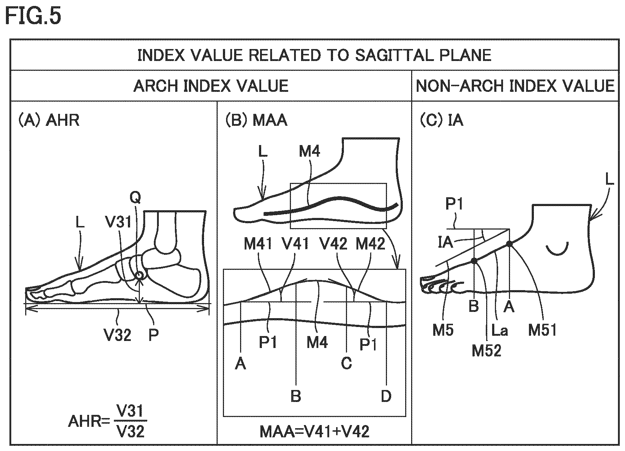

Fig. 5 is a diagram for illustrating the AHR, the MAA, and the IA, each of which is an index value related to a sagittal plane. In Fig. 5, the medial foot-side portion of the right foot of the subject is shown as the foot L.

Fig. 5(A) is a diagram for illustrating the AHR. Fig. 5(A) shows V31 and V32. V31 denotes a distance between a navicular bone tubercle Q and the ground contact surface P of the foot L. V32 denotes a value of the foot length. Further, the AHR denotes a value obtained by dividing V31 by V32 (a ratio of V31 to V32).

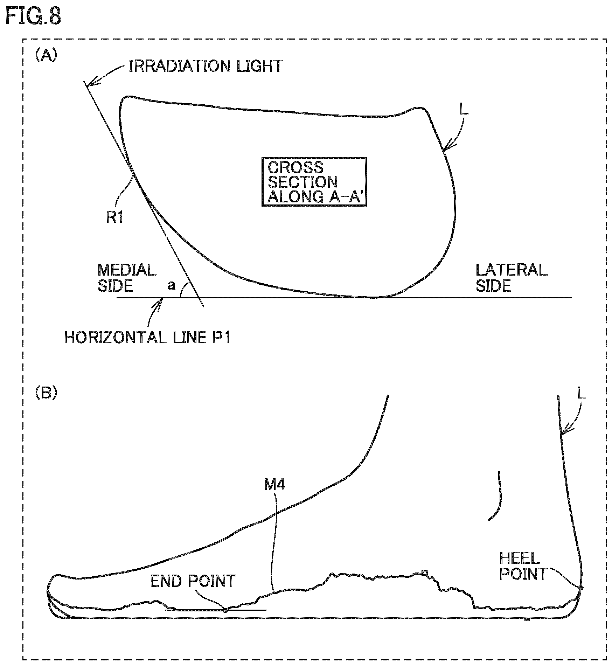

Fig. 5(B) is a diagram for illustrating the MAA. For the MAA, an arch line M4 is used. The arch line M4 is a boundary line of a shadow appearing, for example, when light (irradiation light in Fig. 8(A)) having an inclination at any angle of 45 degrees to 65 degrees is applied from the ground contact surface P to the medial foot-side portion of the foot L. In other words, the arch line M4 is an "apparent arch line" of the foot L.

Further, Fig. 5(B) shows straight lines M41 and M42. The straight line M41 is formed by a front portion of the arch line M4 of the foot L. The front portion of the arch line M4 is a portion extending from a position B at any of 50% to 59% of the foot length to a position A at any of 60% to 70% of the foot length. The "straight line (the straight line M41) formed by the front portion of the arch line M4" is a straight line approximated from a curved line located at the front portion of the arch line M4.

Further, the straight line M42 is formed by a rear portion of the arch line M4 of the foot L. The rear portion of the arch line M4 is a portion extending from a position D at any of 20% to 29% of the foot length to a position C at any of 30% to 40% of the foot length. The "straight line (the straight line M42) formed by the rear portion of the arch line M4" is a straight line approximated from a curved line located at the rear portion of the arch line M4.

Fig. 5(B) shows a front part angle V41 and a rear part angle V42. The front part angle V41 is an angle formed between the straight line M41 and a horizontal line P1. The horizontal line P1 is a straight line parallel to the ground contact surface P. The rear part angle V42 is an angle formed between the straight line M42 and the horizontal line P1. The MAA is calculated by summing the front part angle V41 and the rear part angle V42. In other words, the MAA is a total angle of the front part angle V41 and the rear part angle V42.

Fig. 5(C) is a diagram for illustrating the IA. Fig. 5(C) shows a straight line M5, the horizontal line P1, a point M51, and a point M52. The straight line M5 is formed by an instep part La of the foot L. Specifically, the straight line M5 connects the points M51 and M52. The point M51 is a foot height measurement point. The foot height measurement point is a point located in the instep part La at the position A at any of 50% to 60% of the foot length. The point M52 is the highest point of the first metatarsal head. The highest point of the first metatarsal head is a point located in the instep part La at the position B at any of 70% to 80% of the foot length. The IA is an angle formed between the straight line M5 and the horizontal line P1.

Among the index values related to the sagittal plane described with reference to Fig. 5, the AHH and the MAA each are an example of an arch index value related to the arch of the foot L. Further, the IA is an example of a non-arch index value different from the arch index value.

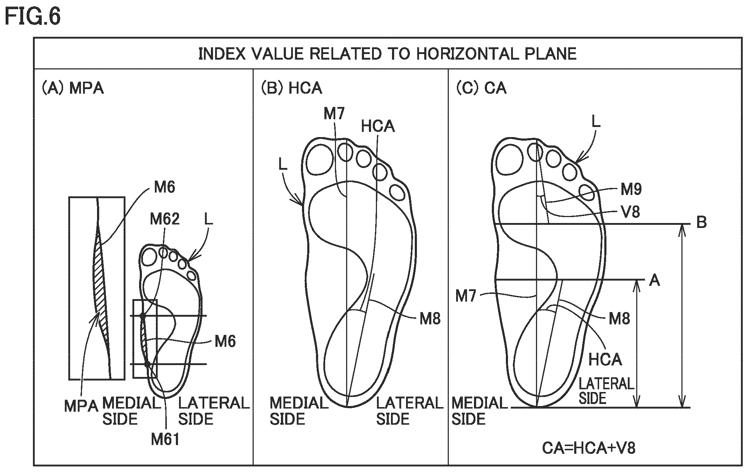

Fig. 6 is a diagram for illustrating the MPA, the HCA, and the CA, each of which is an index value related to a horizontal plane. Fig. 6 shows a sole portion of the right foot of the subject as the foot L. Fig. 6(A) is a diagram for illustrating the MPA. Fig. 6(A) shows a straight line M6.

The straight line M6 connects points M61 and M62. The point M61 is a contour point indicating the heel width of the foot in a plan view along the horizontal plane of the foot. In other words, the point M61 is a heel width measurement point. The heel width measurement point is specifically a point on the contour of the foot L that is located at any of 10% to 20% of the foot length. The point M62 is a contour point on a midfoot portion of the foot. In other words, the contour point on the midfoot portion of the foot is a point on the contour of the foot L that is located at any of 50% to 60% of the foot length.

The MPA is an area of a portion located on the medial side of the foot L from the straight line M6 in a plan view along the horizontal plane of the foot. In Fig. 6(A), this portion is hatched.

Fig. 6(B) is a diagram for illustrating the HCA. Fig. 6(B) shows straight lines M7 and M8. The straight line M7 is a center line of the entire foot L in a plan view along the horizontal plane of the foot L. The straight line M8 is a center line of the rearfoot portion of the foot L. As shown in Fig. 6(C), the rearfoot portion of the foot L corresponds to a portion extending from the heel portion of the foot to the position A at any of 40% to 50% of the foot length. The forefoot portion (describe later) of the foot L corresponds to a portion extending from the heel portion of the foot to the position B at any of 60% to 70% of the foot length. The HCA is an angle formed between the straight lines M7 and M8. The HCA corresponds to a "rearfoot portion angle" of the present disclosure.

Fig. 6(C) is a diagram for illustrating the CA. Fig. 6(C) also shows a straight line M9 in addition to the straight lines M7 and M8. The straight line M9 is a center line of the forefoot portion of the foot L. An angle formed between the straight lines M7 and M9 is defined as an angle V8. The CA is calculated by summing the HCA (see Fig. 6(B)) and the angle V8. In other words, the CA is the total angle of the HCA and the angle V8.

### [Apparent Arch Line]

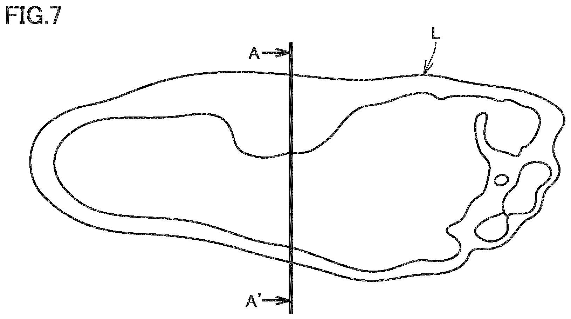

Next, the arch line M4 (an apparent arch line) shown in Fig. 5(B) will be described. In the present embodiment, a cross-sectional view of the foot L is used to illustrate the arch line M4. Fig. 7 is a diagram for illustrating this cross-sectional view. As shown in Fig. 7, in the present embodiment, a cross section of the foot L along a line A-A' (a cross section in the frontal plane) is used.

Fig. 8 is a diagram for illustrating the arch line M4. As shown in Fig. 8(A), R1 denotes a contact point between a line (a medial a-degree line) inclined by "a" degrees toward the medial side of the foot from the ground and the outer shape of the medial side of the foot in the cross section of the foot along the line A-A'. In the present embodiment, "a" is any angle between 45 degrees and 60 degrees. As shown in Fig. 8(B), the contact points R1 are acquired at prescribed intervals (for example, at each 1 mm) in the foot length direction, and a line connecting the plurality of acquired contact points R1 is defined as the arch line M4 in Fig. 5(B).

### [Functional Block Diagram of Evaluation Device]

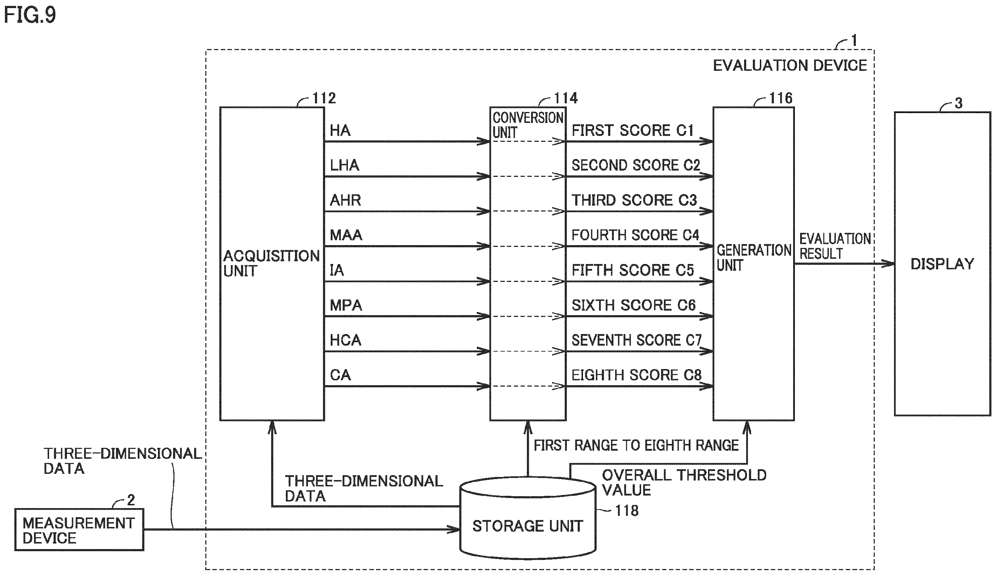

Fig. 9 is a functional block diagram of the evaluation device 1. The evaluation device 1 includes an acquisition unit 112, a conversion unit 114, a generation unit 116, and a storage unit 118. The acquisition unit 112, the conversion unit 114, and the generation unit 116 correspond to the processor 11. The storage unit 118 is implemented by integrating a storage area of the memory 12 and a storage area of the storage 13. The storage unit 118 stores the first range to the eighth range and the overall threshold value that constitute the range group 133 (see Fig. 2).

When the three-dimensional data is transmitted from the measurement device 2 (see also Fig. 1) to the evaluation device 1, the three-dimensional data is temporarily stored in the storage unit 118. Then, the acquisition unit 112 reads the three-dimensional data stored in the storage unit 118 and thereby acquires the three-dimensional data. Further, the acquisition unit 112 computes eight index values from the three-dimensional data. The eight index values are HA, LHA, AHR, MAA, IA, MPA, HCA, and CA described with reference to Figs. 3 and the like. These eight index values are output to the conversion unit 114.

In order to acquire the AHR, it is preferable that the position of the navicular bone tubercle Q (Fig. 5(A)) is specified. The position of the navicular bone tubercle Q may be specified, for example, by marking the position of the navicular bone tubercle Q through conducting palpation and visual inspection of the subject's foot L by an inspector. The acquisition unit 112 calculates the V31 based on the marking and then outputs the AHR. Further, the acquisition unit 112 may estimate the position of the navicular bone tubercle Q, for example, by what is called artificial intelligence (AI). The estimation of the position of the navicular bone tubercle Q is disclosed, for example, in "WO2020/261434A1". Further, the seven index values other than the AHR are acquired by the acquisition unit 112 based on the three-dimensional data without conducting palpation and visual inspection of the subject's foot L by the inspector.

The conversion unit 114 generates and outputs the n-th (n is an integer of 1 to 8) score based on the comparison between the n-th index value and the n-th range. The n-th score includes a first score C1, a second score C2, a third score C3, a fourth score C4, a fifth score C5, a sixth score C6, a seventh score C7, and an eighth score C8. These eight scores are values each indicating the flat foot degree. In the present embodiment, each of the scores is one of a positive value, zero, and a negative value. As the score having a positive value is larger, the foot shape tends to be closer to a flat foot. Further, as the absolute value of the score having a negative value is larger, the foot shape tends to be closer to a flat foot or a cavus foot. The "cavus foot" is a feature of the foot opposite to the flat foot, characterized in that the arch of the sole of the foot L is extremely high. The following describes a specific example of the conversion method by the conversion unit 114.

Further, the case where the n-th (n is an integer of 1 to 8) index value is larger than the n-th range means that the n-th index value is larger than the upper limit value of the n-th range. Further, the case where the n-th (n is an integer of 1 to 8) index value is less than the n-th range means that the n-th index value is smaller than the lower limit value of the n-th range. The "difference between the n-th index value and the n-th range" indicates a difference, for example, between the n-th index value and the center value of the n-th range.

As shown in Fig. 3, the HA is an index value having a tendency that a larger value indicates a shape closer to a flat foot. The conversion unit 114 compares the HA with the first range. When the HA is larger than the first range, the conversion unit 114 outputs a positive first score C1 corresponding to the difference between the HA and the first range. In this case, the "positive n-th score C corresponding to the difference" indicates that the positive n-th score increases as the difference increases.

When the HA is less than the first range, the conversion unit 114 outputs a negative first score C1 corresponding to the difference between the HA and the first range. In this case, the "negative n-th (n is an integer of 1 to 8) score C corresponding to the difference" indicates that the negative n-th score decreases as the difference increases. When the HA belongs to the first range, the conversion unit 114 outputs zero as the first score C1.

Further, the LHA is an index value having a tendency that a larger value indicates a shape closer to a flat foot. The conversion unit 114 compares the LHA with the second range. When the LHA is larger than the second range, the conversion unit 114 outputs a positive second score C2 corresponding to the difference between the LHA and the second range. Further, when the LHA is less than the second range, the conversion unit 114 outputs a negative second score C2 corresponding to the difference between the LHA and the second range. When the LHA belongs to the second range, the conversion unit 114 outputs zero as the second score C2.

Further, the AHR is an index value having a tendency that a smaller value indicates a shape closer to a flat foot. The conversion unit 114 compares the AHR with the third range. Then, when the AHR is less than the third range, the conversion unit 114 outputs a positive third score C3 corresponding to the difference between the AHR and the third range. Further, when the AHR is larger than the third range, the conversion unit 114 outputs a negative third score C3 corresponding to the difference between the AHR and the third range. When the AHR belongs to the third range, the conversion unit 114 outputs zero as the third score C3.

Further, the MAA is an index value having a tendency that a smaller value indicates a shape closer to a flat foot. The conversion unit 114 compares the MAA with the fourth range. Then, when the MAA is less than the fourth range, the conversion unit 114 outputs a positive fourth score C4 corresponding to the difference between the MAA and the fourth range. Further, when the MAA is larger than the fourth range, the conversion unit 114 outputs a negative fourth score C4 corresponding to the difference between the MAA and the fourth range. When the MAA belongs to the fourth range, the conversion unit 114 outputs zero as the fourth score C4.

Further, the IA is an index value having a tendency that a smaller value indicates a shape closer to a flat foot. The conversion unit 114 compares the IA with the fifth range. Then, when the IA is less than the fifth range, the conversion unit 114 outputs a positive fifth score C5 corresponding to the difference between the IA and the fifth range. Further, when the IA is larger than the fifth range, the conversion unit 114 outputs a negative fifth score C5 corresponding to the difference between the IA and the fifth range. When the IA belongs to the fifth range, the conversion unit 114 outputs zero as the fifth score C5.

Further, the MPA is an index value having a tendency that a larger value indicates a shape closer to a flat foot. The conversion unit 114 compares the MPA with the sixth range. Then, when the MPA is larger than the sixth range, the conversion unit 114 outputs a positive sixth score C6 corresponding to the difference between the MPA and the sixth range. When the MPA is less than the sixth range, the conversion unit 114 outputs a negative sixth score C6 corresponding to the difference between the MPA and the sixth range. When the MPA belongs to the sixth range, the conversion unit 114 outputs zero as the sixth score C6.

Further, the HCA is an index value having a tendency that a smaller value indicates a shape closer to a flat foot. The conversion unit 114 compares the HCA with the seventh range. Then, when the HCA is less than the seventh range, the conversion unit 114 outputs a positive seventh score C7 corresponding to the difference between the HCA and the seventh range. When the HCA is larger than the seventh range, the conversion unit 114 outputs a negative seventh score C7 corresponding to the difference between the HCA and the seventh range. When the HCA belongs to the seventh range, the conversion unit 114 outputs zero as the seventh score C7.

Further, the CA is an index value having a tendency that a smaller value indicates a shape closer to a flat foot. The conversion unit 114 compares the CA with the eighth range. Then, when the CA is less than the eighth range, the conversion unit 114 outputs a positive eighth score C8 corresponding to the difference between the CA and the eighth range. When the CA is larger than the eighth range, the conversion unit 114 outputs a negative eighth score C8 corresponding to the difference between the CA and the eighth range. When the CA belongs to the eighth range, the conversion unit 114 outputs zero as the eighth score C8.

The first score C1 to the eighth score C8 output from the conversion unit 114 are input to the generation unit 116. The generation unit 116 calculates a total score that is a total value of the first score C1 to the eighth score C8. The total score corresponds to a flat-foot level of the present disclosure. Then, the generation unit 116 compares the total score with the overall threshold value. The center value of the overall threshold value is, for example, zero. When the total score is larger than the overall threshold value, the generation unit 116 evaluates that the subject's foot L is a flat foot, and then generates evaluation information indicating this evaluation. When the total score is equal to or less than the overall threshold value, the generation unit 116 evaluates that the subject's foot L is not a flat foot, and generates evaluation information indicating this evaluation.

After generating the evaluation result, the generation unit 116 outputs the data indicating the evaluation result to the display 3. The display 3 shows an image based on the data.

### [Reason for Selection of Eight Index Values]

The following describes the reason why the inventors have selected the above-mentioned eight index values in order to determine whether or not the subject's foot L is a flat foot. In order to determine whether or not the subject's foot L is a flat foot, there is a method of irradiating the foot L with X-rays (which will be hereinafter also referred to as an "X-ray irradiation method"). This X-ray irradiation method is a highly accurate method as a method of evaluating whether or not the subject's foot is a flat foot. In this X-ray irradiation method, seven X-ray index values are defined. Theses seven X-ray index values are disclosed, for example, in the following Documents 1 and 2.

Document 1 is "Correlation of Foot Posture Index With Plantar Pressure and Radiographic Measurements in Pediatric Flatfoot" by Lee, J. S., Kim, K. B., Jeong, J. O., Kwon, N. Y., Jeong, S. M., M. (Annals of Rehabilitation Medicine 39(1), 10-17 (2015), DOI: 10.5535/arm.2015.39.1.10).

Document 2 is "A protocol for classifying normal and flat arched foot posture for research studies using clinical and radiographic measurements" by Murley G. S. S., Menz, H. B Landorf1, K. B. (Journal of Foot and Ankle Research 2009, 2 22, 2009 DOI: 10.1186/1757 1146 2 22).

The above-mentioned seven X-ray index values include an X-ray index value in the frontal plane, an X-ray index value in the sagittal plane, and an X-ray index value in the horizontal plane. The X-ray index value in the frontal plane is a heel tibia angle (HTA). The X-ray index values in the sagittal plane are a calcaneal pitch (CP), a calcaneal first metatarsal angle (C1MA), a Meary's angle (MA), and a navicular index (NI). Further, the X-ray index values in the horizontal plane are a talo-navicular coverage angle (TNCA) and a talo-second metatarsal angle (T2MA).

In this case, these seven X-ray index values are considered as being accurate in evaluating whether or not the subject's foot L is a flat foot. However, all of the seven X-ray index values are angles related to foot bones, and are index values based on the premise that the subject's foot is irradiated with X-rays. Thus, the subject's foot needs to be irradiated with X-rays in order to use these seven X-ray index values, and this may cause a problem that a burden such as X-ray irradiation on the subject increases.

Accordingly, the inventors have examined index values highly correlated with seven X-ray index values among the index values that can be computed from the three-dimensional data. As a result of the examination, the inventors have found the above-mentioned eight index values.

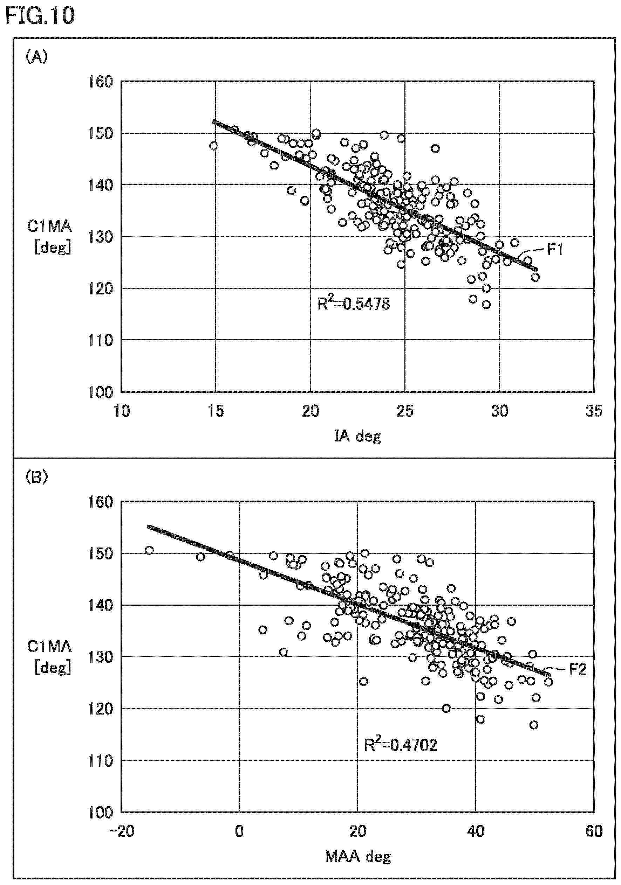

Fig. 10 is a diagram showing a correlation between the index values in the present embodiment and the X-ray index values. Fig. 10(A) is a diagram showing a correlation between the C1MA as an X-ray index value and the IA as an index value in the present embodiment. In the example in Fig. 10(A), a plurality of plots and a regression line F1 for the plurality of plots are shown. The example in Fig. 10(A) shows a negative correlation and indicates that the square value (a determination coefficient) of a correlation coefficient R is 0.5478. Note that the method of calculating the correlation coefficient in the example in Fig. 10 is directed to a plurality of feet. Further, the correlation coefficient (the determination coefficient) in Fig. 10 was calculated by using an equation in which the "covariance between the index value in the present embodiment and the X-ray index value" was divided by the "value obtained by multiplication of the standard deviation of the index values in the present embodiment and the standard deviation of the X-ray index values", and then, the value obtained as a result of this division was squared.

Fig. 10(B) is a diagram showing a correlation between the C1MA as an X-ray index value and the MAA as an index value in the present embodiment. The example in Fig. 10(B) shows a plurality of plots and a regression line F2 for the plurality of plots. The example in Fig. 10(B) shows a negative correlation and indicates that the square value of the correlation coefficient R is 0.4702.

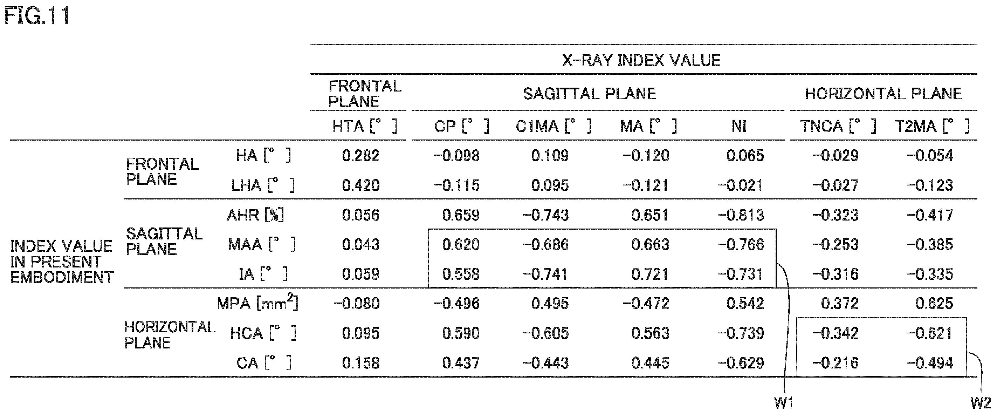

Fig. 11 is a diagram of an examples showing correlation coefficients between eight index values in the present embodiment and seven X-ray index values. For example, in Fig. 11, the correlation coefficient R between the HA as an index value in the present embodiment and the HTA as an X-ray index value is 0.282.

As shown in Fig. 11, index values whose correlation coefficients with the X-ray index values were high to some extent were selected by the inventors as the eight index values in the present embodiment. In this case, as indicated by a frame W1 in Fig. 11, it is shown that the correlation coefficients for the MAA and the IA are relatively higher than those in the case of other index values. Further, as indicated by a frame W2 in Fig. 11, it is shown that the correlation coefficients for the HCA and the CA are relatively high at the angle of the horizontal plane.

### [For Center Values of Respective Ranges]

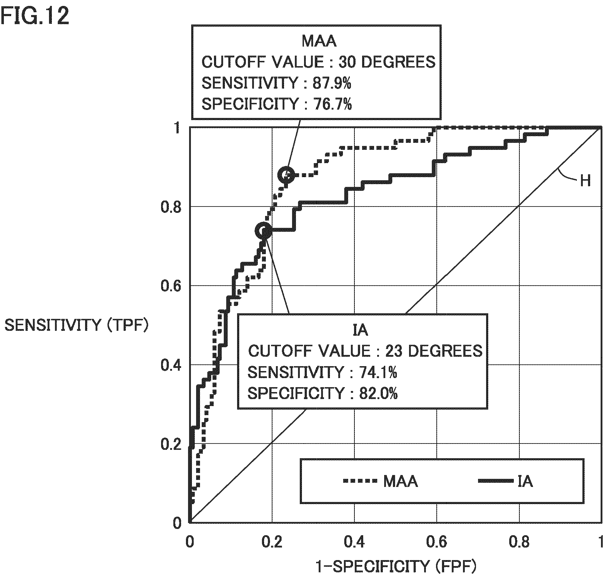

Hereinafter, the center values of the first range to the eighth range are also referred to as a first threshold value to an eighth threshold value. The following describes a method of determining the first to the eighth threshold values. Fig. 12 is a diagram showing examples of receiver operating characteristic (ROC) curves of the above-mentioned MAA and the IA having high correlation coefficients. The ROC curve of the IA is shown with a solid line and the ROC curve of the MAA is shown with a dashed line. Note that the ROC curves were created for a plurality of feet.

With regard to the ROC curves in Fig. 12, the vertical axis represents sensitivity (true positive fraction: TPF) while the horizontal axis represents 1-specificity (false positive fraction: FPF). Further, Fig. 12 shows a straight line H along which the TPF and the FPF have the same value. Among the plots constituting each ROC curve, a plot indicating the maximum distance to the straight line H (hereinafter also referred to as a "maximum plot") is a plot corresponding to a highly accurate threshold value (a cutoff value).

In the example in Fig. 12, the maximum plot of the IA shows a sensitivity of 74.1%, a specificity of 82%, and a cutoff value of 23 degrees. This cutoff value of 23 degrees is adopted as a threshold value of the IA (the fifth threshold value in Fig. 3). Further, the maximum plot of the MAA shows a sensitivity of 87.9%, a specificity of 76.7%, and a cutoff value of 30 degrees. This cutoff value of 30 degrees is adopted as the threshold value of the MAA (the fourth threshold value in Fig. 3).

Further, the inventors also created ROC curves for other index values (HA, LHA, AHR, MPA, HCA, and CA), and determined threshold values corresponding to these other index values.

### [Flowchart]

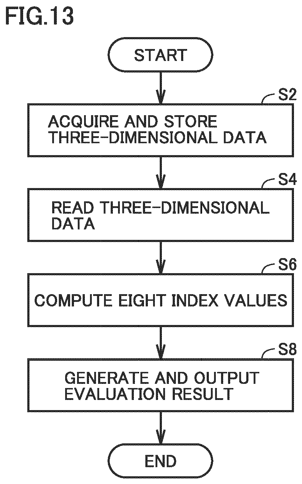

Fig. 13 is a flowchart illustrating a main process of the evaluation device 1. First, in step S2, the evaluation device 1 acquires three-dimensional data from the measurement device 2 and temporarily stores the three-dimensional data in the memory 12 (the storage unit 118 in Fig. 9).

Then, in step S4, the evaluation device 1 reads the three-dimensional data from the memory 12. In step S6, the evaluation device 1 computes (acquires) eight index values from the three-dimensional data that has been read. Then, in step S8, the evaluation device 1 generates an evaluation result based on the eight index values and outputs the generated evaluation result to the display 3. The display 3 shows an image based on the result.

### [Proposal Result]

Next, the proposal result shown in Fig. 1 will be described. In the case of employing a configuration in which the evaluation device 1 outputs the proposal result, the evaluation device 1 computes specific index values other than the above-mentioned eight index values from the three-dimensional data. For example, the specific index values are a foot length, a shape of a finger, and the like. Based on the eight index values and the specific index values, the evaluation device 1 detects at least one of: a shoe to be fitted to the subject; a shoe last (last) to be fitted to the subject; a sockliner to be fitted to the subject; foot care suitable for the subject; training suitable for the subject; and the like. Then, the evaluation device 1 outputs the detected details as a proposal result to the display 3. The display 3 shows a proposed image based on the proposal result.

The measuring person visually checks the proposed image and proposes a product based on the proposed image to the subject. For example, the proposed image is an image showing shoes to be fitted to the subject. The measuring person makes a proposal to the subject about purchase of the shoes.

### [General Overview]

In the method of irradiating the subject's foot with X-rays to evaluate whether or not the subject's foot is a flat foot (the X-ray irradiation method), the subject's foot needs to be irradiated with X-rays. This may cause a problem that a burden such as X-ray irradiation on the subject increases. Thus, the evaluation device 1 in the present embodiment computes eight types of index values from the three-dimensional data measured by the measurement device, and then, evaluates the flat-foot level (for example, the above-mentioned total score) of the subject's foot based on the index values. Therefore, since there is no need to irradiate the subject with X-rays, the evaluation device 1 can evaluate the flat-foot level of the subject's foot while it can suppress a burden on the subject.

Further, in the X-ray irradiation method, an inspector visually checks an X-ray radiograph created by the X-ray irradiation to inspect whether or not the subject's foot is a flat foot. This however may cause a problem that the inspector may be limited to some specialists such as doctors. On the other hand, according to the evaluation device 1 in the present embodiment, the measuring person visually checks the evaluation result and thereby can specify whether or not the subject's foot is a flat foot. Therefore, not only some few specialists but also general measuring persons can specify whether or not the subject's foot is a flat foot.

In the X-ray irradiation method, the accuracy in inspecting whether or not the subject's foot is a flat foot depends on the quality of an X-ray radiograph. On the other hand, since the evaluation device 1 in the present embodiment performs an evaluation about a flat foot without using an X-ray radiograph, it allows the measuring person to perform a highly reproducible evaluation. Further, the X-ray irradiation method may depend on the inspection skill of the inspector. On the other hand, since the evaluation device 1 in the present embodiment allows a general measuring person to specify an evaluation result, it allows the measuring person to perform a highly reproducible evaluation.

Further, as described with reference to Figs. 5 and the like, the index value related to the sagittal plane includes an arch index value related to the arch of the foot (the AHR and the MAA in Fig. 5) and a non-arch index value (the IA in Fig. 5) different from the arch index value.

In general, whether or not the subject's foot is a flat foot is attributed to the arch structure of the foot. Thus, it is conceivable to provide a configuration in which whether or not the subject's foot is a flat foot is evaluated using only the arch index value but without using the non-arch index value. However, for example, in the case of performing the evaluation using only the arch index value, there may be a specific subject who is determined to have a flat foot but actually does not have a flat foot. Such a specific subject is, for example, a person having a foot skeleton, foot bones, muscles of the foot sole, and the like that are well developed more than those of a person having an ordinary foot. In the configuration in which only the arch index value is used to evaluate whether or not the subject's foot is a flat foot, there is a concern that the evaluation accuracy may be degraded since the evaluation result may indicate that such a specific subject has a flat foot even though this specific subject actually does not have a flat foot.

Thus, the evaluation device 1 in the present embodiment uses not only the arch index value but also the non-arch index value to evaluate whether or not the subject's foot L is a flat foot. Therefore, the evaluation device 1 can perform multifaceted evaluations. Accordingly, even when the subject is a specific subject as described above, the evaluation device 1 can make an accurate evaluation about a flat foot.

The non-arch index value also includes IA. As indicated by frame W1 in Fig. 11, the IA is an index value having a large correlation coefficient with a highly accurate X-ray index value. Therefore, the evaluation device 1 can perform a highly accurate evaluation by making an evaluation about a flat foot with use of the non-arch index value including the IA.

Further, the arch index value includes MAA. The MAA is an index value having a large correlation coefficient with a highly accurate X-ray index value, as indicated by frame W1 in Fig. 11. Therefore, the evaluation device 1 can perform a highly accurate evaluation by making an evaluation about a flat foot with use of the arch index value including the MAA.

The arch index value includes AHR. The AHR is an arch height ratio and indicates an index value that is directly attributed to whether or not the subject's foot is a flat foot. Therefore, the evaluation device 1 can perform a highly accurate evaluation by making an evaluation about a flat foot with use of the arch index value including the AHR.

Further, the index value related to the horizontal plane includes HCA. As indicated by frame W2 in Fig. 11, the HCA is an index value having a relatively large correlation coefficient with a highly accurate X-ray index value. Therefore, the evaluation device 1 can perform a highly accurate evaluation by making an evaluation about a flat foot with use of the index value including the HCA.

The index value related to the horizontal plane includes CA. The CA is an index value having a relatively large correlation coefficient with a highly accurate X-ray index value, as indicated by frame W2 in Fig. 11. Therefore, the evaluation device 1 can perform a highly accurate evaluation by making an evaluation about a flat foot with use of the index value including the CA.

The index value related to the horizontal plane includes MPA. As described also with reference to Fig. 6(A), the physical quantity of the MPA is an area. Therefore, since the evaluation device 1 makes an evaluation about a flat foot based on the area that is a physical quantity different from the angle, it can perform multifaceted evaluations.

The index value related to the frontal plane includes HA. Therefore, the evaluation device 1 can perform an evaluation about a flat foot based on the index value related to the frontal plane. Further, the index value related to the frontal plane includes LHA. Therefore, the evaluation device 1 can perform an evaluation about a flat foot based on the index value related to the frontal plane.

Further, based on the above-mentioned eight index values, the evaluation device 1 proposes at least one of a shoe for the subject, a shoe last for the subject, and a sockliner of the shoe for the subject. Therefore, the evaluation device 1 can propose a shoe, a shoe last, and a sockliner in which the evaluation about a flat foot for the subject's foot has been reflected. In this way, the evaluation device 1 of the present disclosure can apply the eight index values (or the flat-foot level) not only to the evaluation about a flat foot but also to industrial techniques such as making a proposal to the subject.

### <Second Embodiment>

The first embodiment has been described above with regard to a configuration in which the flat-foot level is information indicating whether or not the subject's foot is a flat foot. The flat-foot level in the second embodiment is defined as information quantitatively indicating a feature of a foot.

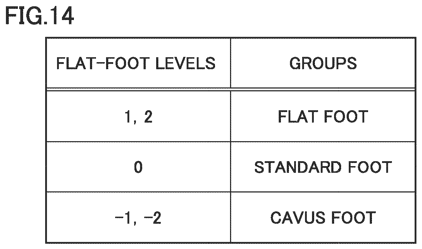

Fig. 14 is an example of a table showing a correspondence relation between the flat-foot levels and groups. In the example in Fig. 14, the flat-foot levels are quantitatively shown in five stages (-2, -1, 0, 1, 2). In addition to a group of flat feet, the groups in Fig. 14 include a group of cavus feet (high arch) mentioned above and a group of standard feet. The "standard foot" indicates a feature of a foot not corresponding to a flat foot and a cavus foot. Note that this table is stored, for example, in the storage unit 118 (see Fig. 9).

In the example in Fig. 14, a flat-foot level of 1 or 2 is associated with the group of flat feet. Further, a flat-foot level of 0 is associated with the group of standard feet. Further, a flat-foot level of -1 or -2 is associated with the group of cavus feet.

The generation unit 116 converts the above-described total score into a flat-foot level in five stages. Further, the generation unit 116 refers to the table in Fig. 14 to specify the group corresponding to each flat-foot level. Then, the generation unit 116 generates an evaluation result corresponding to each group and causes the display 3 to show the evaluation result. For example, when the flat-foot level is -1 or -2, the evaluation device 1 causes the display 3 to show a character image stating, "You have cavus feet". When the flat-foot level is 0, the evaluation device 1 causes the display 3 to show a character image stating, "You have standard feet".

According to such a configuration, the evaluation device 1 not only can evaluate whether or not the subject's foot is a flat foot, but also can evaluate whether the subject's foot is a standard foot or a cavus foot. Therefore, the evaluation device 1 can more specifically evaluate the subject's foot.

As a modification, the flat foot group may be further divided. For example, a flat-foot level of "1" may be classified as a "low flat foot group" and a flat-foot level of "2" may be classified as a "high flat foot group". Also, the cavus foot group may be further divided. For example, a flat-foot level of "-1" may be classified as a "low cavus foot group", and a flat-foot level of "2" may be classified as a "high cavus foot group".

As a modification, the three groups into which the foot shape is classified may be any one group or any two groups. In the present embodiment, the flat-foot level is a concept including at least one of a level indicating a flat foot, a level indicating a cavus foot, and a level indicating a standard foot.

### <Third Embodiment>

In the third embodiment, the calculation of the above-mentioned n-th score will be described in detail. In the example described above with reference to Fig. 3, the first range to the eighth range are applied to the eight index values. In the present embodiment, other preferable examples (the first prescribed range, the second prescribed range, and the third prescribed range) of the first to the eighth ranges will be described.

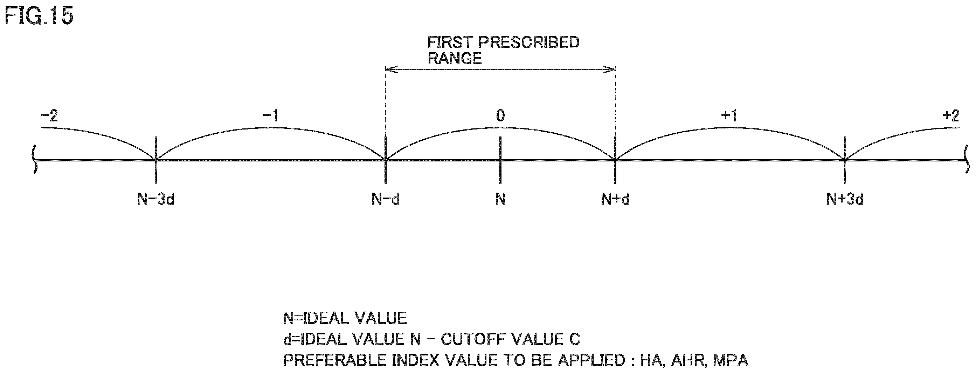

Fig. 15 is a diagram for illustrating the first prescribed range. The center value (a reference value) of the first prescribed range is an ideal value N of the first value, which will be described later. In this case, the ideal value N is a value that is ideal (not biased) in terms of the skeleton shape of the foot. For example, the ideal value N of the index value (HA, LHA) in the frontal plane is a value obtained when varus/valgus of the calcaneus bone is in an ideal state, i.e., obtained when neither varus nor valgus occurs. Further, the ideal value N of the index value (AHR, MAA, IA) in the sagittal plane is a value obtained when the arch height is in an ideal state, i.e., obtained when the foot has neither a high arch nor a low arch. In addition, the ideal value N of the index value (MPA, HCA, CA) in the horizontal plane is a value obtained when the curve of the foot is in an ideal state, i.e., obtained when the foot is not in an excessively curved or straight state.

Further, the lower limit value of the first prescribed range is a value obtained by subtracting d from N (N-d). The upper limit value of the first prescribed range is a value obtained by adding d to N (N+d). Further, "d" is a value calculated by subtracting a cutoff value C of the first value V1 from the ideal value of a first value V1. The first value V1 will be described later.

In the present embodiment, the preferable index value to which the first prescribed range is applied is at least one of the HA, the AHR, and the MPA. At least one of the HA, the AHR, and the MPA corresponds to the "first value" of the present disclosure. The first value is also referred to as the "first value V1".

It is preferable to apply the HA, the AHR, and the MPA for the first prescribed range since the ideal value N of each of the HA, the AHR, and the MPA is easily derived. Specifically, it is preferable to apply the HA to the first prescribed range since the measured values of the HA extend over the positive and negative values, and thus, the ideal value N can be set at 0 degrees.

Further, it is preferable to apply the AHR to the first prescribed range since there is a cutoff value for each of the high arch and the low arch, and thus, the ideal value N can be set at the average value of the values for the high arch and the low arch. For example, when the cut-off value for the high arch is 20 degrees and the cut-off value for the low arch is 10 degrees, the ideal value N of the AHR is 15 degrees.

Further, it is preferable to apply the MPA to the first prescribed range since the measured values of the MPA extend over the positive and negative values, and thus, the ideal value N can be set at 0 mm².

For example, when the acquisition unit 112 (see Fig. 9) acquires the first value V1 (at least one of the HA, the AHR, and the MPA), the flat-foot level is evaluated based on whether or not the first value V1 belongs to the first prescribed range.

The following first describes the case where the first value V1 is the HA. As described with reference to Fig. 3, the HA is an index value having a tendency that a larger value indicates a shape closer to a flat foot. In the example in Fig. 15, when the first value V1 is less than N-3d, the conversion unit 114 sets the first score C1 at "-2". When the first value V1 is equal to or greater than N-3d and is less than N-d, the conversion unit 114 sets the first score C1 at "-1".

When the first value V1 is equal to or greater than N-d and is less than N+d (belongs to the first prescribed range), the conversion unit 114 sets the first score C1 at "0". When the first value V1 is equal to or greater than N+d and is less than N+3d, the conversion unit 114 sets the first score C1 at "+1". When the first value V1 is equal to or greater than N+3d, the conversion unit 114 sets the first score C1 at "+2".

When the first value V1 is the AHR, the scores in Fig. 15, i.e., -2, -1, +1, and +2 result in +2, +1, -1, and -2, respectively. When the first value V1 is the MPA, the scores are the same as those in Fig. 15.

As described above, since the evaluation device 1 sets the reference in the first prescribed range as the ideal value of the first value V1, the flat-foot level can be evaluated based on the skeleton shape of the foot without being influenced, for example, by the parameters of the population of the subjects. Since the first value V1 is at least one of the HA, the AHR, and the MPA, the evaluation device 1 can apply the first prescribed range defined based on the ideal value as a reference to the index value (the HA, the AHR, and the MPA) from which the ideal value is easily derived.

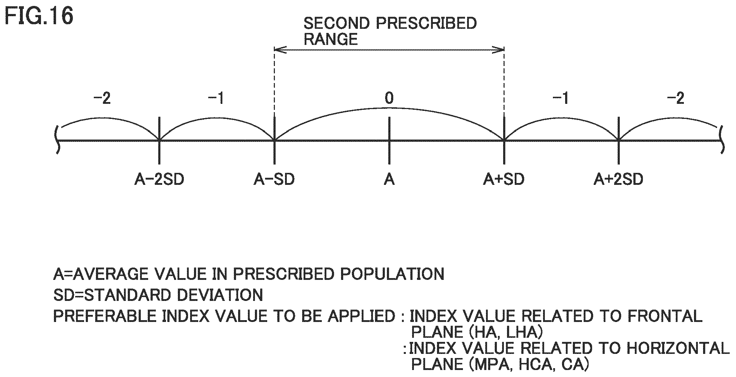

Fig. 16 is a diagram for illustrating the second prescribed range. The center value (a reference value) of the second prescribed range is a previously calculated average value A of the second values (described later) in a prescribed population. The population is, for example, a group constituted of a certain number of (for example, 200) subjects. Further, the lower limit value of the second prescribed range is a value obtained by subtracting SD from the average value A (average value A-SD). The upper limit value of the second prescribed range is a value obtained by adding SD to the average value A (average value A+SD). Also, the "SD" indicates the standard deviation in the above-mentioned population. In other words, the second prescribed range is a range conforming to a normal distribution.

In the present embodiment, the preferable index value to which the second prescribed range is applied is at least one of the index value related to the frontal plane and the index value related to the horizontal plane. At least one of the index value related to the frontal plane and the index value related to the horizontal plane corresponds to the "second value" of the present disclosure. The second value is also referred to as a "second value V2". Note that the index value related to the frontal plane is at least one of the HA and the LHA, as shown in Fig. 3. The index value related to the horizontal plane is at least one of the MPA, the HCA, and the CA, as shown in Fig. 3.

The following describes the reason why it is preferable to apply the second prescribed range for at least one of the index value related to the frontal plane and the index value related to the horizontal plane. The inventors have found that the index value related to the frontal plane and the index value related to the horizontal plane were low in discrimination ability for a flat foot (area under the curve (AUC)). Thus, according to such a configuration, the evaluation device 1 can apply the second prescribed range not employing a cut-off value for the index value with a low discrimination ability for a flat foot (AUC).

For example, when the acquisition unit 112 (see Fig. 9) acquires the second value V2 (at least one of the index value related to the frontal plane and the index value related to the horizontal plane), the flat-foot level is evaluated based on whether or not the second value V2 belongs to the second prescribed range.

The following first describes the case where the second value V2 is the HA. As described with reference to Fig. 3, the HA is an index value having a tendency that a larger value indicates a shape closer to a flat foot. In the example in Fig. 16, when the second value V2 is less than A-2SD, the conversion unit 114 sets the first score C1 at "-2". When the second value V2 is equal to or greater than A-2SD and is less than A-SD, the conversion unit 114 sets the first score C1 at "-1".

When the second value V2 is equal to or greater than A-SD and is less than A+SD (belongs to the second prescribed range), the conversion unit 114 sets the first score C1 at "0". When the second value V2 is equal to or greater than A+SD and is less than A+2SD, the conversion unit 114 sets the first score C1 at "+1". When the second value V2 is equal to or greater than A+2SD, the conversion unit 114 sets the first score C1 at "+2".

In the case where the second value V2 is the HCA or the CA (the index value having a tendency that a smaller value indicates a shape closer to a flat foot), the scores in Fig. 16, i.e., -2, -1, +1, and +2 result in +2, +1, -1, and -2, respectively. When the second value V2 is the LHA or the MPA, the scores are the same as those in Fig. 16.

As described above, the second prescribed range is defined by adding or subtracting the standard deviation SD to or from the upper limit value and the lower limit value of the second prescribed range, assuming that the reference is the average value of the second values V2 in the population. Therefore, since the evaluation device 1 evaluates the flat-foot level according to the normal distribution, it can evaluate where the subject is located in the normal distribution. Further, the evaluation device 1 can apply the second prescribed range not employing a cut-off value to each of the index values (the index value related to the frontal plane and the index value related to the horizontal plane) with a low discrimination ability for a flat foot (AUC).

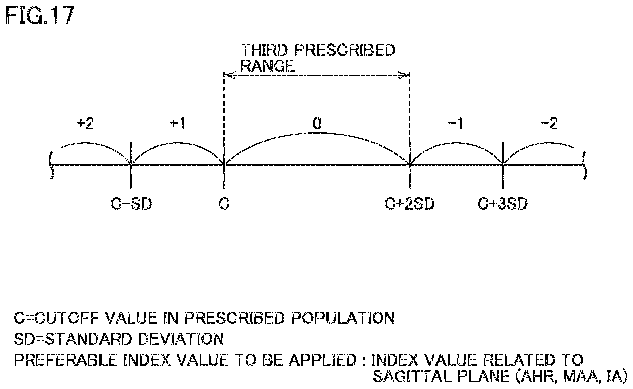

Fig. 17 is a diagram for illustrating the third prescribed range. In the third prescribed range, the cutoff value C of the third value (described later) in a prescribed population is defined as a reference value. The population is, for example, a group constituted of a certain number of (for example, 200) subjects. Further, the lower limit value of the third prescribed range is the cutoff value C. The upper limit value of the third prescribed range is a value obtained by adding 2SD to the cutoff value C (cutoff value C+2SD). Further, "SD" indicates the standard deviation of the third values in the above-mentioned population.

In the present embodiment, the preferable index value to which the third prescribed range is applied is the index value related to the sagittal plane. The index value related to the sagittal plane corresponds to the "third value" of the present disclosure. The third value is also referred to as a "third value V3". Note that the index value related to the sagittal plane is at least one of the AHR, the MAA, and the IA, as shown in Fig. 3.

The following describes the reason why it is preferable to apply the third prescribed range for the index value related to the sagittal plane. The inventors have found that the index value related to the sagittal plane was high in discrimination ability for a flat foot (AUC). Thus, according to such a configuration, the evaluation device 1 can improve the accuracy in determination about a flat foot by applying the third prescribed range defined based on the cut-off value as a reference to the index value (the index value related to the sagittal plane) with a high discrimination ability for a flat foot.

For example, when the acquisition unit 112 (see Fig. 9) acquires the third value V3 (the index value related to the sagittal plane), the flat-foot level is evaluated based on whether or not the third value V3 belongs to the third prescribed range.

The following first describes the case where the third value V3 is the AHR. As described with reference to Fig. 3, the AHR is an index value having a tendency that a smaller value indicates a shape closer to a flat foot. In the example in Fig. 17, when the third value V3 is less than C-SD, the conversion unit 114 sets the third score C3 at "+2". When the third value V3 is equal to or greater than C-SD and is less than C, the conversion unit 114 sets the third score C3 at "+1".

When the third value V3 is equal to or greater than C and is less than C+2SD (belongs to the third prescribed range), the conversion unit 114 sets the third score C3 at "0". When the third value V3 is equal to or greater than C+2SD and is less than C+3SD, the conversion unit 114 sets the third score C3 at "-1". When the third value V3 is equal to or greater than C+3SD, the conversion unit 114 sets the third score C3 at "-2". When the third value V3 is the MAA or the IA, the scores are the same as those in Fig. 17.

As described above, the third prescribed range is defined based on the premise that the reference is defined as the cutoff value C for the third value V3 in the population. Therefore, since the evaluation device 1 evaluates the flat-foot level based on the cut-off value C, it can evaluate the flat-foot level based on the skeleton shape of the foot while also considering the variation in the distribution of the population of the subjects. Further, since the evaluation device 1 applies the third prescribed range employing the cut-off value to the index value (the index value related to the sagittal plane) with a high discrimination ability for a flat foot, the accuracy in evaluation of the flat-foot level can be improved.

In at least one of the above-mentioned first to third prescribed ranges, the value to be added to or subtracted from the reference value may be another value. For example, for the first prescribed range, a value to be added or subtracted may be the standard deviation SD on condition that the ideal value N is set as the center value. Further, in the third prescribed range, the cutoff value C may be set as a center value.

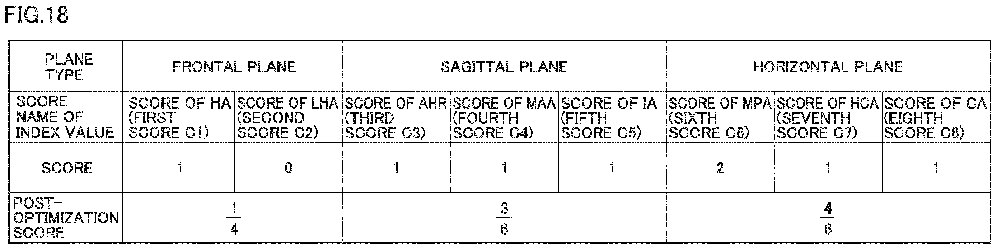

Fig. 18 is an example showing scores and the like calculated with use of the above-mentioned first to third prescribed ranges for each of the eight index values. Fig. 18 shows an example in which the first score C1 to the eighth score C8 are "1", "0", "1", "1", 1", "2", "1", and "1".

In the example in Fig. 18, the post-optimization scores are shown. The post-optimization scores are calculated for each plane (a frontal plane, a sagittal plane, and a horizontal plane) of the foot. The post-optimization scores are typically normalized scores. The post-optimization scores are calculated, for example, by dividing, for each plane, the total value of the scores on each plane by the maximum total value on each plane. For example, the post-optimization score of the sagittal plane is calculated by 3 (= 1 + 1 + 1) ÷ 6 (= 2 × 3).

Further, the generation unit 116 calculates the flat-foot level by substituting the post-optimization score of each plane into the following equation (1). 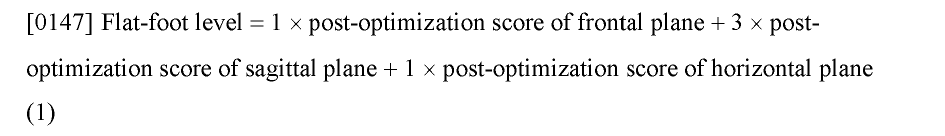Flat-foot level = 1 × post-optimization score of frontal plane + 3 × post-optimization score of sagittal plane + 1 × post-optimization score of horizontal plane (1)

In the present disclosure, the post-optimization score of the sagittal plane in Fig. 18 corresponds to the "first index value score" of the present disclosure. Further, each of the post-optimization score of the frontal plane and the post-optimization score of the horizontal plane in Fig. 18 corresponds to the "second index value score" of the present disclosure.

In the equation (1), the first coefficient multiplied by the post-optimization score of the sagittal plane (the first index value score) is "3". On the other hand, in the equation (1), the second coefficient multiplied by each of the post-optimization score of the frontal plane and the post-optimization score of the horizontal plane (the second index value score) is "1". Thus, the first coefficient is larger than the second coefficient. As described above, the generation unit 116 in the present embodiment evaluates (calculates) the flat-foot level with a weight placed more on the first index value score than on the second index value score. Therefore, the evaluation device 1 can evaluate the flat-foot level with emphasis placed more on the sagittal plane highly correlated with the flat foot than on the frontal plane and the horizontal plane. In the case where each of the scores in Fig. 18 is substituted into the above-mentioned equation (1), the flat-foot level is about 4.8.

### <Fourth Embodiment>

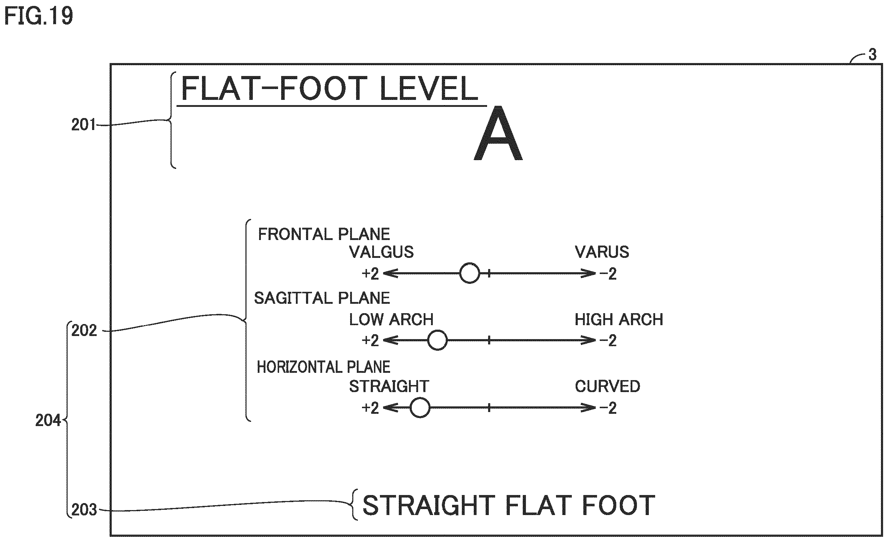

In the fourth embodiment, the display screen on the display 3 will be described in detail. Fig. 19 is a diagram for illustrating an example of the display screen on the display 3. In the example in Fig. 19, a flat-foot level image 201 and a flat-foot type image 204 are displayed. The flat-foot level image 201 is an image showing a flat-foot level. The generation unit 116 calculates the flat-foot level, for example, with use of the above-mentioned equation (1). In the example in Fig. 19, a symbol "A" corresponding to the flat-foot level is displayed.

The flat-foot type image 204 is an image showing a flat-foot type. The following describes the flat-foot type. The flat-foot type is typically information generated based on at least two index values among three index values including: an index value related to the frontal plane; an index value related to the sagittal plane; and an index value related to the horizontal plane. It is preferable that the at least two index values include the index value related to the sagittal plane highly correlated with a flat foot. In the present embodiment, the flat-foot type is generated using all of the at least two index values.

The flat-foot type is more specifically the information defined by the features of each of the three index values. The features about the index value related to the frontal plane are valgus and varus. The features about the index value related to the sagittal plane are a low arch and a high arch. The features about the index value related to the horizontal plane are a straight state and a curved state.

The flat-foot type image 204 includes a feature image 202 and a name image 203. The feature image 202 relates to the feature of each of the three index values described above. The name image 203 shows the name of a flat-foot type.

The flat-foot type in the example in Fig. 19 is information generated when the scores are as shown in the example in Fig. 18. In the example in Fig. 18, the post-optimization score of the frontal plane is +1/4. Accordingly, the feature image 202 shows an image indicating the level closer to valgus as a feature of the frontal plane. Further, in the example in Fig. 18, the post-optimization score of the sagittal plane is +3/6. Accordingly, the feature image 202 shows an image indicating the level closer to a low arch as a feature of the sagittal plane. Further, in the example in Fig. 18, the post-optimization score of the sagittal plane is +4/6. Accordingly, the feature image 202 shows an image indicating the level closer to a straight state as a feature of the sagittal plane.

Further, as the name image 203, a character image of a "straight flat foot" is displayed.

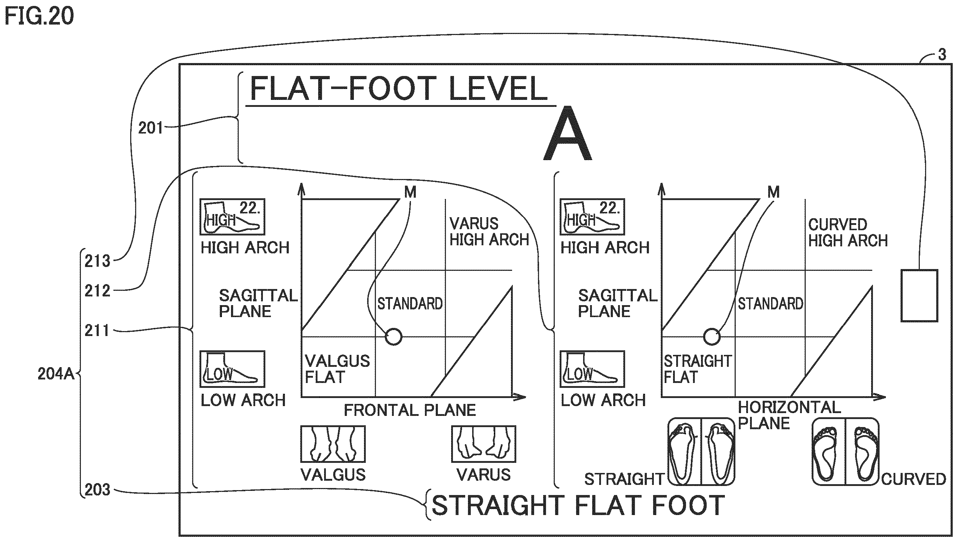

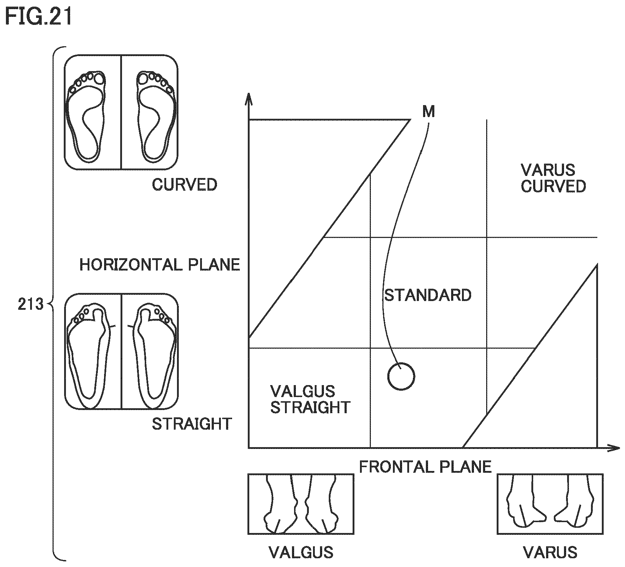

Fig. 20 is a diagram for illustrating an example of a flat-foot type image 204A or the like as another flat-foot type image. The flat-foot type image 204A includes a first coordinate image 211, a second coordinate image 212, and a third coordinate image 213. Fig. 20 shows the third coordinate image 213 that is replaced with a square image. Fig. 21 is a diagram showing an example of the third coordinate image 213.

The first coordinate image 211, the second coordinate image 212, and the third coordinate image 213 are images in which the features of two index values among the above-mentioned three index values are represented in a plane defined by an X axis and a Y axis. In the first coordinate image 211, the X axis corresponds to the feature of the frontal plane, and the Y axis corresponds to the feature of the sagittal plane. In the second coordinate image 212, the X axis corresponds to the feature of the horizontal plane, and the Y axis corresponds to the feature of the sagittal plane. In the third coordinate image 213, the X axis corresponds to the feature of the frontal plane, and the Y axis corresponds to the feature of the horizontal plane. In each of the first coordinate image 211, the second coordinate image 212, and the third coordinate image 213, an image M shows an index value specified in the subject. Based on this image M, a subject who has visually checked the first coordinate image 211, the second coordinate image 212, and the third coordinate image 213 can recognize the features in the above-mentioned three index values for the subject.

As described above, the evaluation device 1 in the present embodiment can evaluate the flat-foot type based on at least two of the index values related to the frontal plane, the sagittal plane, and the horizontal plane. Note that a flat foot typically indicates the state in which the foot arch generally lowers (a morphological change on the sagittal plane). Thus, the evaluation device 1 evaluates at least one of the horizontal plane and the frontal plane about the lowered arch and thereby can perform multifaceted evaluations. In other words, the evaluation device 1 can estimate what caused such a lowered arch. Thus, it is preferable that the above-mentioned at least two index values include an index value related to the sagittal plane for evaluating whether or not the arch lowers. Therefore, the evaluation device 1 can evaluate the flat-foot type in a multifaceted manner.

Further, the evaluation device 1 causes the display 3 to show the flat-foot level image 201 in Figs. 19 and 20 and the flat-foot type image 204 that indicates the features of the above-mentioned at least two index values. Therefore, the evaluation device 1 allows the subject to recognize his/her flat-foot level and his/her flat-foot type.

The examples in Figs. 20 and 21 have been described with regard to the screen on which two index values among the index values in the three planes are two-dimensionally displayed. However, the display 3 may three-dimensionally show the index values in the three planes.

### <Fifth Embodiment>

In general, the skeleton of the foot is different depending on the attribute of the subject. For example, the skeleton of the foot is different between Japanese females and American males. Thus, when the evaluation device 1 employs the same prescribed range for the same index value in the case where the subject is a Japanese female and the case where the subject is an American male, the accuracy in evaluation of the flat-foot level may not be able to be improved.

Thus, the evaluation device 1 in the fifth embodiment evaluates the flat-foot level of the subject with use of the prescribed range corresponding to the attributes of the subject. The attributes of the subject include, for example, gender, race, height, weight, age, and the like.

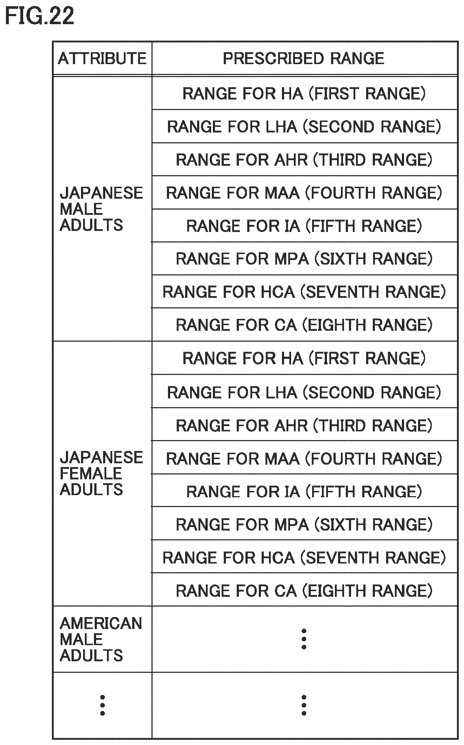

Fig. 22 is an example of a database (DB) defining the correspondences between the attributes and the ranges applied to the index values. The storage unit 118 in the fifth embodiment stores the DB. The range includes, for example, the first range to the eighth range (see Fig. 3). This DB corresponds to "range information" of the present disclosure.

In the example in Fig. 22, the first to eighth ranges corresponding to Japanese adult males (attribute) are stored, and the first to eighth ranges corresponding to Japanese adult females (attribute) are stored. The ranges corresponding to other attributes are not described.

Next, a flow of the process of the evaluation device 1 in the present embodiment will be described. The acquisition unit 112 acquires the attribute of the subject. For example, the evaluation device 1 displays an attribute input screen (not shown) and causes the subject to input his/her attribute through the input screen, and then, the acquisition unit 112 acquires the attribute of the subject. Then, the conversion unit 114 refers to the DB in Fig. 22 to specify a prescribed range corresponding to the acquired attribute. Further, the flat-foot level is evaluated based on whether or not at least one index value belongs to the specified prescribed range. For example, the method in the third embodiment is used as an evaluation method.

The evaluation device 1 in the present embodiment evaluates the flat-foot level based on whether or not at least one index value belongs to a prescribed range corresponding to the attribute of the subject. Therefore, the evaluation device 1 can improve the accuracy in evaluation of the flat-foot level as compared with the configuration in which the same prescribed range is used irrespective of the attribute of the subject.

When the prescribed range in Fig. 22 includes a range conforming to the normal distribution (see Fig. 16), it is possible to evaluate where the subject is located in the normal distribution corresponding to the attribute of the subject.

### <Modifications>

(1) The embodiments have been described above with regard to the evaluation device 1 that performs an evaluation about a flat foot with use of the eight index values described with reference to Fig. 11. However, the evaluation device 1 may perform an evaluation about a flat foot with use of some of the eight index values. For example, the evaluation device 1 may perform an evaluation about a flat foot with use of one of the eight index values.
   Further, for example, the evaluation device 1 may use the index value of one of the HA and the LHA shown in Fig. 4. Further, the evaluation device 1 may use the index value of one of the HCA and the CA shown in Fig. 4. Further, the evaluation device 1 may perform an evaluation about a flat foot with use of the MAA, the IA, the HCA, and the CA, each of which is an index value having a high degree of correlation with the X-ray index value.
(2) The embodiments have been described above with regard to a configuration in which three devices (the measurement device 2, the evaluation device 1, and the display 3) are separate devices, as shown in Fig. 1. However, a configuration in which at least two of the three devices are the same device may be employed.
(3) In the configuration adopted in each of the above-described embodiments, the evaluation about a flat foot is performed based on the comparison between the eight index values and the ranges corresponding to the eight index values, as shown in Fig. 9. However, the evaluation device 1 may perform an evaluation about a flat foot based on the eight index values with use of what is called AI.
(4) The embodiments have been described above with regard to a configuration in which the evaluation device 1 evaluates the flat-foot level of the subject's foot based on at least one index value. However, the evaluation device 1 may be configured to classify the shape of the arch of the subject's foot into any one of a plurality of groups based on at least one index value.
(5) The embodiments have been described above with regard to a configuration in which the evaluation result is output to the display. However, the evaluation result may be output to other destinations. A configuration in which the evaluation result is output, for example, to a printer may be employed. When such a configuration is employed, the evaluation device 1 causes a printer to output a sheet of paper on which the evaluation result is printed.

Further, at least two of the configurations in the above-described present embodiments and the configurations described in the modifications may be implemented in combination.

According to such a configuration, the flat-foot level can be evaluated based on the attribute of the subject.

It should be understood that the embodiments and the modifications thereof disclosed herein are illustrative and non-restrictive in every respect. The scope of the present invention is defined by the claims, rather than the description above, and is intended to include any modifications within the scope of the claims.

### REFERENCE SIGNS LIST

1 evaluation device, 2 measurement device, 3 display, 11 processor, 12 memory, 13 storage, 14 interface, 17 processor bus, 18 removable disk, 21 top plate, 22 laser measurement unit, 100 evaluation system, 112 acquisition unit, 114 conversion unit, 116 generation unit, 118 storage unit, 131 evaluation program, 133 range group, 201 flat-foot level image, 202 feature image, 203 name image, 204, 204A flat-foot type image, 211 first coordinate image, 212 second coordinate image, 213 third coordinate image.

## Claims

1. An evaluation device (1) that evaluates a shape of a foot of a subject, the evaluation device (1) comprising:
a memory (12) that stores three-dimensional data of the shape of the foot of the subject measured by a measurement device (2); and
a processor (11) that computes an index value specifying the shape of the foot from the three-dimensional data read from the memory (12), wherein
the processor (11) is configured to
compute, from the three-dimensional data, at least one index value among an index value related to a frontal plane of the foot, an index value related to a sagittal plane of the foot, and an index value related to a horizontal plane of the foot, the at least one index value being computed as the index value specifying the shape of the foot, and
evaluate a flat-foot level of the foot of the subject based on the at least one index value,
**characterized in that**
the index value related to the sagittal plane includes
an arch index value related to an arch of the foot, and
a non-arch index value different from the arch index value, and
the processor (11) is configured to compute at least the index value related to the sagittal plane of the foot from the three-dimensional data,
wherein
the non-arch index value includes an angle between a straight line formed by an instep part of the foot and a straight line formed by a horizontal line of the foot.

2. The evaluation device (1) according to claim 1, wherein
the arch index value includes a total angle of:
an angle formed between a straight line formed by a front portion of an arch line of the foot and a horizontal line of the foot; and
an angle formed between a straight line formed by a rear portion of the arch line and the horizontal line of the foot.

3. The evaluation device (1) according to claim 1, wherein the arch index value includes an arch height ratio.

4. The evaluation device (1) according to claim 1, wherein
the index value related to the horizontal plane of the foot includes a rearfoot portion angle formed between a center line of an entirety of the foot and a center line of a rearfoot portion of the foot, and
the processor (11) is configured to compute at least the index value related to the horizontal plane of the foot from the three-dimensional data.

5. The evaluation device (1) according to claim 4, wherein
the index value related to the horizontal plane of the foot includes a total angle of:
an angle formed between the center line of the entirety of the foot and a center line of a forefoot portion of the foot; and
the rearfoot portion angle.

6. The evaluation device (1) according to claim 1, wherein
the index value related to the horizontal plane of the foot includes an area of a portion located on a medial side of the foot from a straight line connecting a contour point indicating a heel width of the foot and a contour point of a midfoot portion of the foot in a plan view along the horizontal plane of the foot, and
the processor (11) is configured to compute at least the index value related to the horizontal plane of the foot from the three-dimensional data.

7. The evaluation device (1) according to claim 1, wherein
the index value related to the frontal plane of the foot includes an angle formed between a vertical axis of the foot and a heel midline of the foot, and
the processor (11) is configured to compute at least the index value related to the frontal plane of the foot from the three-dimensional data.

8. The evaluation device (1) according to claim 1, wherein
the index value related to the frontal plane of the foot includes an angle formed between a leg axis of the foot and a heel midline of the foot, and
the processor (11) is configured to compute at least the index value related to the frontal plane of the foot from the three-dimensional data.

9. The evaluation device (1) according to claim 1, wherein the processor (11) is configured to classify a shape of an arch of the foot of the subject into one of a plurality of groups based on an evaluation result of the flat-foot level.

10. The evaluation device (1) according to claim 9, wherein the plurality of groups include at least two of a group indicating a flat foot, a group indicating a cavus foot, and a group indicating a standard foot.

11. The evaluation device (1) according to claim 1, wherein the processor (11) is configured to, based on the at least one index value, make at least one of proposals of: a shoe for the subject; a shoe last for the subject; a sockliner of the shoe for the subject; foot care for the subject; and training for the subject.

12. The evaluation device (1) according to claim 1, wherein
the processor (11) is configured to
compute, from the three-dimensional data, at least two index values among the index value related to the frontal plane of the foot, the index value related to the sagittal plane of the foot, and the index value related to the horizontal plane of the foot, and
evaluate a flat-foot type of the foot of the subject based on the at least two index values.

13. The evaluation device (1) according to claim 12, further comprising a display (3) that shows an image (201) indicating the flat-foot level and an image (204A) indicating the flat-foot type, wherein
the image (204A) indicating the flat-foot type includes information about features of the at least two index values.

14. The evaluation device (1) according to claim 1, wherein
the processor (11) is configured to
specify a first index value score about the index value related to the sagittal plane of the foot, and a second index value score about at least one index value among the index value related to the frontal plane of the foot and the index value related to the horizontal plane of the foot, and
evaluate the flat-foot level with a weight placed more on the first index value score than on the second index value score.

15. The evaluation device (1) according to claim 1, wherein
the at least one index value includes a first value,
the processor (11) is configured to evaluate the flat-foot level based on whether or not the first value belongs to a first prescribed range, and
the first prescribed range is defined based on an ideal value of the first value as a reference.

16. The evaluation device (1) according to claim 15, wherein
the first value includes at least one of:
as the index value related to the frontal plane, an angle formed between a vertical axis of the foot and a heel midline of the foot;
as the index value related to the sagittal plane, an arch height ratio; and
as the index value related to the horizontal plane, an area of a portion located on a medial side of the foot from a straight line connecting a contour point indicating a heel width of the foot and a contour point of a midfoot portion of the foot in a plan view along the horizontal plane of the foot.

17. The evaluation device (1) according to claim 1, wherein
the at least one index value includes a second value,
the processor (11) is configured to evaluate the flat-foot level based on whether or not the second value belongs to a second prescribed range, and
the second prescribed range is defined based on, as a reference, an average value of the second values in a prescribed population.

18. The evaluation device (1) according to claim 17, wherein
the second value includes at least one of:
the index value related to the frontal plane; and
the index value related to the horizontal plane.

19. The evaluation device (1) according to claim 1, wherein
the at least one index value includes a third value,
the processor (11) is configured to evaluate the flat-foot level based on whether or not the third value belongs to a third prescribed range, and
the third prescribed range is defined based on, as a reference, a cutoff value of the third value in a prescribed population.

20. The evaluation device (1) according to claim 19, wherein the third value includes at least one value included in the index value related to the sagittal plane.

21. The evaluation device (1) according to claim 1, wherein
the memory (12) is configured to store range information in which a prescribed range corresponding to the at least one index value is defined in association with each attribute of the subject, and
the processor (11) is configured to
acquire the attribute of the subject,
refer to the range information to specify the prescribed range corresponding to the acquired attribute and corresponding to the at least one index value, and
evaluate the flat-foot level based on whether or not the at least one index value belongs to the specified prescribed range.

22. An evaluation method of evaluating a shape of a foot of a subject, the evaluation method comprising:
computing, from three-dimensional data of the shape of the foot of the subject measured by a measurement device (2), at least one index value among an index value related to a frontal plane of the foot, an index value related to a sagittal plane of the foot, and an index value related to a horizontal plane of the foot; and
evaluating a flat-foot level of the foot of the subject based on the at least one index value,
**characterized in that**
the index value related to the sagittal plane includes
an arch index value related to an arch of the foot, and
a non-arch index value different from the arch index value, and
at least the index value related to the sagittal plane of the foot from the three-dimensional data is computed,
wherein
the non-arch index value includes an angle between a straight line formed by an instep part of the foot and a straight line formed by a horizontal line of the foot.

## Patentansprüche

1. Bewertungsvorrichtung (1), die eine Form eines Fußes einer Testperson bewertet, wobei die Bewertungsvorrichtung (1) umfasst:
einen Speicher (12), der von einer Messvorrichtung (2) gemessene dreidimensionale Daten der Form des Fußes der Testperson speichert; und
einen Prozessor (11), der aus den aus dem Speicher (12) ausgelesenen dreidimensionalen Daten einen Indexwert berechnet, der die Form des Fußes spezifiziert, wobei
der Prozessor (11) eingerichtet ist zum
Berechnen, aus den dreidimensionalen Daten, mindestens eines Indexwertes von einem Indexwert, der auf eine Frontalebene des Fußes bezogen ist, einem Indexwert, der auf eine Sagittalebene des Fußes bezogen ist, und einem Indexwert, der auf eine Horizontalebene des Fußes bezogen ist, wobei der mindestens eine Indexwert als der die Form des Fußes spezifizierende Indexwert berechnet ist, und
Bewerten eines Plattfuß-Grades des Fußes der Testperson auf Grundlage des mindestens einen Indexwerts,
**dadurch gekennzeichnet, dass**
der Indexwert, der auf die Sagittalebene bezogen ist, aufweist
einen Gewölbe-Indexwert, der auf ein Gewölbe des Fußes bezogen ist, und
einen Nicht-Gewölbe-Indexwert, der sich von dem Gewölbe-Indexwert unterscheidet, und
der Prozessor (11) eingerichtet ist, um aus den dreidimensionalen Daten mindestens den Indexwert zu berechnen, der auf die Sagittalebene des Fußes bezogen ist,
wobei
der Nicht-Gewölbe-Indexwert einen Winkel zwischen einer geraden Linie, die durch einen Spann-Teil des Fußes gebildet wird, und einer geraden Linie, die durch eine horizontale Linie des Fußes gebildet wird, aufweist.

2. Bewertungsvorrichtung (1) gemäß Anspruch 1, wobei
Der Gewölbe-Indexwert einen Gesamtwinkel aufweist aus:
einem Winkel, der zwischen einer geraden Linie, die durch einen vorderen Abschnitt einer Gewölbelinie des Fußes gebildet wird, und einer horizontalen Linie des Fußes gebildet wird; und
einem Winkel, der zwischen einer geraden Linie, die durch einen hinteren Abschnitt der Gewölbelinie gebildet wird, und der horizontalen Linie des Fußes gebildet wird.

3. Bewertungsvorrichtung (1) gemäß Anspruch 1, wobei der Gewölbe-Indexwert ein Gewölbehöhenverhältnis aufweist.

4. Bewertungsvorrichtung (1) gemäß Anspruch 1, wobei
der Indexwert, der auf die Horizontalebene des Fußes bezogen ist, einen Hinterfußabschnittswinkel aufweist, der zwischen einer Mittellinie einer Gesamtheit des Fußes und einer Mittellinie eines Hinterfußabschnitts des Fußes gebildet wird, und
der Prozessor (11) eingerichtet ist, um aus den dreidimensionalen Daten mindestens den Indexwert zu berechnen, der auf die Horizontalebene des Fußes bezogen ist.

5. Bewertungsvorrichtung (1) gemäß Anspruch 4, wobei
der Indexwert, der auf die Horizontalebene des Fußes bezogen ist, einen Gesamtwinkel aufweist aus:
einem Winkel, der zwischen der Mittellinie der Gesamtheit des Fußes und einer Mittellinie eines Vorfußabschnitts des Fußes gebildet wird; und
dem Hinterfußabschnittswinkel.

6. Bewertungsvorrichtung (1) gemäß Anspruch 1, wobei
der Indexwert, der auf die Horizontalebene des Fußes bezogen ist, ein Gebiet eines Abschnitts aufweist, der sich auf einer medialen Seite des Fußes von einer geraden Linie befindet, die einen Konturpunkt, der eine Fersenbreite des Fußes angibt, und einen Konturpunkt eines Mittelfußabschnitts des Fußes in einer Draufsicht entlang der Horizontalebene des Fußes verbindet, und
der Prozessor (11) eingerichtet ist, um aus den dreidimensionalen Daten mindestens den Indexwert berechnet, der auf die Horizontalebene des Fußes bezogen ist.

7. Bewertungsvorrichtung (1) gemäß Anspruch 1, wobei
der Indexwert, der auf die Frontalebene des Fußes bezogen ist, einen Winkel aufweist, der zwischen einer vertikalen Achse des Fußes und einer Fersenmittellinie des Fußes gebildet wird, und
der Prozessor (11) eingerichtet ist, um aus den dreidimensionalen Daten zumindest den Indexwert zu berechnen, der auf die Frontalebene des Fußes bezogen ist.

8. Bewertungsvorrichtung (1) gemäß Anspruch 1, wobei
der Indexwert, der auf die Frontalebene des Fußes bezogen ist, einen Winkel aufweist, der zwischen einer Beinachse des Fußes und einer Fersenmittellinie des Fußes gebildet wird, und
der Prozessor (11) eingerichtet ist, um aus den dreidimensionalen Daten zumindest den Indexwert zu berechnen, der auf die Frontalebene des Fußes bezogen ist.

9. Bewertungsvorrichtung (1) gemäß Anspruch 1, wobei der Prozessor (11) eingerichtet ist, um eine Form eines Gewölbes des Fußes der Testperson auf Grundlage eines Bewertungsergebnisses des Plattfuß-Grades in eine einer Vielzahl von Gruppen zu klassifizieren.

10. Bewertungsvorrichtung (1) gemäß Anspruch 9, wobei die Vielzahl von Gruppen mindestens zwei aus einer Gruppe, die einen Plattfuß anzeigt, einer Gruppe, die einen Hohlfuß anzeigt, und einer Gruppe, die einen Standardfuß anzeigt, aufweist.

11. Bewertungsvorrichtung (1) gemäß Anspruch 1, wobei der Prozessor (11) eingerichtet ist, um auf Grundlage des mindestens einen Indexwerts mindestens eines der Vorschläge macht aus: Einem Schuh für die Testperson; einem Schuhleisten für die Testperson; einer Einlegesohle des Schuhs für die Testperson; Fußpflege für die Testperson; und Training für die Testperson.

12. Bewertungsvorrichtung (1) gemäß Anspruch 1, wobei
der Prozessor (11) eingerichtet ist zum
Berechnen aus den dreidimensionalen Daten mindestens zweier Indexwerte aus dem Indexwert, der auf die Frontalebene des Fußes bezogen ist, dem Indexwert, der auf die Sagittalebene des Fußes bezogen ist, und dem Indexwert, der auf die Horizontalebene des Fußes bezogen ist, und
Bewerten eines Plattfuß-Typ des Fußes der Testperson auf Grundlage von mindestens zwei Indexwerten.

13. Bewertungsvorrichtung (1) gemäß Anspruch 12, das ferner eine Anzeige (3) umfasst, die ein Bild (201), das den Plattfuß-Grad angibt und ein Bild (204A), das den Plattfuß-Typ angibt, zeigt, wobei
das Bild (204A), das den Plattfuß-Typ angibt, Informationen über Merkmale der mindestens zwei Indexwerte aufweist.

14. Bewertungsvorrichtung (1) gemäß Anspruch 1, wobei
der Prozessor (11) eingerichtet ist zum
Spezifizieren eines ersten Indexwert-Scores über den Indexwert, der auf die Sagittalebene des Fußes bezogen ist, und eines zweiten Indexwert-Scores über mindestens einen Indexwert aus dem Indexwert, der auf die Frontalebene des Fußes bezogen ist und dem Indexwert, der auf die Horizontalebene des Fußes bezogen ist, und
Bewerten des Plattfuß-Grades, mit einer stärkeren Gewichtung auf dem ersten Indexwert-Score als auf dem zweiten Indexwert-Score.

15. Bewertungsvorrichtung (1) gemäß Anspruch 1, wobei
der mindestens eine Indexwert einen ersten Wert aufweist,
der Prozessor (11) eingerichtet ist, um den Plattfuß-Grad auf der Grundlage davon zu bewerten, ob der erste Wert zu einem ersten vorgeschriebenen Bereich gehört oder nicht, und
der erste vorgeschriebene Bereich auf Grundlage eines Idealwerts des ersten Werts als Referenz definiert ist.

16. Bewertungsvorrichtung (1) gemäß Anspruch 15, wobei
der erste Wert mindestens eines aufweist aus:
Als den Indexwert, der auf die Frontalebene bezogen ist, einem Winkel, der zwischen einer vertikalen Achse des Fußes und einer Fersenmittellinie des Fußes gebildet wird;
als den Indexwert, der auf die Sagittalebene bezogen ist, einem Gewölbehöhenverhältnis; und
als den Indexwert, der auf die Horizontalebene bezogen ist, einem Gebiet eines Abschnitts, der sich auf einer medialen Seite des Fußes von einer geraden Linie befindet, die einen Konturpunkt, der eine Fersenbreite des Fußes angibt, und einen Konturpunkt eines Mittelfußabschnitts des Fußes in einer Draufsicht entlang der Horizontalebene des Fußes verbindet.

17. Bewertungsvorrichtung (1) gemäß Anspruch 1, wobei
der mindestens eine Indexwert einen zweiten Wert aufweist,
der Prozessor (11) eingerichtet ist, um den Plattfuß-Grad auf Grundlage davon zu bewerten, ob der zweite Wert zu einem zweiten vorgeschriebenen Bereich gehört oder nicht, und
der zweite vorgeschriebene Bereich auf Grundlage eines Durchschnittswerts der zweiten Werte in einer vorgeschriebenen Population als Referenz definiert ist.

18. Bewertungsvorrichtung (1) gemäß Anspruch 17, wobei
der zweite Wert mindestens eines aufweist aus:
dem Indexwert, der auf die Frontalebene bezogen ist; und
dem Indexwert, der auf die Horizontalebene bezogen ist.

19. Bewertungsvorrichtung (1) gemäß Anspruch 1, wobei
der mindestens eine Indexwert einen dritten Wert umfasst,
der Prozessor (11) eingerichtet ist, um den Plattfuß-Grad auf Grundlage davon zu bewerten, ob der dritte Wert zu einem dritten vorgeschriebenen Bereich gehört oder nicht, und
der dritte vorgeschriebene Bereich auf der Grundlage eines Cut-off-Werts des dritten Werts in einer vorgeschriebenen Population als Referenz definiert ist.

20. Bewertungsvorrichtung (1) gemäß Anspruch 19, wobei der dritte Wert mindestens einen Wert aufweist, der in dem Indexwert, der auf die Sagittalebene bezogen ist, enthalten ist.

21. Bewertungsvorrichtung (1) gemäß Anspruch 1, wobei
der Speicher (12) eingerichtet ist, um Bereichsinformationen zu speichern, in denen ein vorgegebener Bereich, der dem mindestens einen Indexwert entspricht, in Verbindung mit jedem Attribut der Testperson definiert ist, und
der Prozessor (11) eingerichtet ist zum
Erfassen des Attributs der Testperson,
Beziehen auf die Bereichsinformationen, um den vorgeschriebenen Bereich zu spezifizieren, der dem erfassten Attribut entspricht und der dem mindestens einen Indexwert entspricht, und
Bewerten des Plattfuß-Grades auf Grundlage davon, ob der mindestens eine Indexwert zu dem spezifizierten vorgeschriebenen Bereich gehört oder nicht.

22. Bewertungsverfahren eines Bestimmens einer Form eines Fußes einer Textperson, wobei das Bewertungsverfahren umfasst:
Berechnen, aus von einem Messgerät (2) gemessenen dreidimensionalen Daten der Form des Fußes der Testperson, mindestens eines Indexwerts aus einem Indexwert, der auf die Frontalebene des Fußes bezogen ist, einem Indexwert, der auf die Sagittalebene des Fußes bezogen ist und einem Indexwert, der auf die Horizontalebene des Fußes bezogen ist; und
Bewertung eines Plattfuß-Grades des Fußes der Testperson auf Grundlage des mindestens einen Indexwerts,
**dadurch gekennzeichnet, dass**
der Indexwert, der auf die Sagittalebene bezogen ist, aufweist
einen Gewölbe-Indexwert, der auf ein Gewölbe des Fußes bezogen ist, und
einen Nicht-Gewölbe-Indexwert, der sich von dem Gewölbe-Indexwert unterscheidet, und
zumindest der Indexwert, der auf die Sagittalebene des Fußes bezogen ist aus den dreidimensionalen Daten berechnet wird,
wobei
der Nicht-Gewölbe-Indexwert einen Winkel zwischen einer geraden Linie, die durch einen Spann-Teil des Fußes gebildet wird, und einer geraden Linie, die durch eine horizontale Linie des Fußes gebildet wird, aufweist.

## Revendications

1. Dispositif d'évaluation (1) qui évalue une forme d'un pied d'un sujet, le dispositif d'évaluation (1) comprenant :
une mémoire (12) qui stocke des données tridimensionnelles de la forme du pied du sujet, mesurées par un dispositif de mesure (2) ; et
un processeur (11) qui calcule une valeur d'indice spécifiant la forme du pied à partir des données tridimensionnelles lues de la mémoire (12), dans lequel
le processeur (11) est configuré pour
calculer, à partir des données tridimensionnelles, au moins une valeur d'indice parmi une valeur d'indice liée à un plan frontal du pied, une valeur d'indice liée à un plan sagittal du pied et une valeur d'indice liée à un plan horizontal du pied, l'au moins une valeur d'indice étant calculée comme la valeur d'indice spécifiant la forme du pied, et
évaluer un niveau de pied plat du sujet sur la base de l'au moins une valeur d'indice,
**caractérisé en ce que**
la valeur d'indice liée au plan sagittal comporte
une valeur d'indice de voûte liée à une voûte du pied, et
une valeur d'indice de non-voûte différente de la valeur d'indice de voûte, et
le processeur (11) est configuré pour calculer, à partir des données tridimensionnelles, au moins la valeur d'indice liée au plan sagittal du pied,
dans lequel
la valeur d'indice de non-voûte comporte un angle entre une ligne droite formée par une part de coup-de-pied du pied et une ligne droite formée par une ligne horizontale du pied.

2. Dispositif d'évaluation (1) selon la revendication 1, dans lequel
la valeur d'indice de voûte comporte à un angle total de :
un angle formé entre une ligne droite formée par une partie antérieure d'une ligne de voûte du pied et une ligne horizontale du pied ; et
un angle formé entre une ligne droite formée par une partie arrière de la ligne de voûte et la ligne horizontale du pied.

3. Dispositif d'évaluation (1) selon la revendication 1, dans lequel la valeur d'indice de voûte comporte un rapport de hauteur de voûte.

4. Dispositif d'évaluation (1) selon la revendication 1, dans lequel
la valeur d'indice liée au plan horizontal du pied comporte un angle de partie d'arrière pied formé entre une ligne de centre d'un ensemble du pied et une ligne de centre d'une partie d'arrière pied du pied, et
le processeur (11) est configuré pour calculer, à partir des données tridimensionnelles, au moins la valeur d'indice liée au plan horizontal du pied.

5. Dispositif d'évaluation (1) selon la revendication 4, dans lequel
la valeur d'indice liée au plan horizontal du pied comporte un angle total de :
un angle formé entre la ligne de centre de l'ensemble du pied et une ligne de centre d'une partie d'avant pied du pied ; et
l'angle de partie d'arrière pied.

6. Dispositif d'évaluation (1) selon la revendication 1, dans lequel
la valeur d'indice liée au plan horizontal du pied comporte une zone d'une partie située à un côté médial du pied, d'une ligne droite reliant un point de contour indiquant une largeur de talon du pied et un point de contour d'une partie de milieu du pied, dans une vue en plan le long du plan horizontal du pied, et
le processeur (11) est configuré pour calculer, à partir des données tridimensionnelles, au moins la valeur d'indice liée au plan horizontal du pied.

7. Dispositif d'évaluation (1) selon la revendication 1, dans lequel
la valeur d'indice reliée au plan frontal du pied comporte un angle formé entre un axe vertical du pied et une ligne médiane du talon, et
le processeur (11) est configuré pour calculer, à partir des données tridimensionnelles, au moins la valeur d'indice reliée au plan frontal du pied.

8. Dispositif d'évaluation (1) selon la revendication 1, dans lequel
la valeur d'indice reliée au plan frontal du pied comporte un angle formé entre un axe de jambe du pied et une ligne médiane du talon du pied, et
le processeur (11) est configuré pour calculer, à partir des données tridimensionnelles, au moins la valeur d'indice reliée au plan frontal du pied.

9. Dispositif d'évaluation (1) selon la revendication 1, dans lequel le processeur (11) est configuré pour classer une forme d'une voûte du pied du sujet dans un d'une pluralité de groupes, sur la base d'un résultat d'évaluation du niveau de pied plat.

10. Dispositif d'évaluation (1) selon la revendication 9, dans lequel la pluralité de groupes comporte au moins deux parmi un groupe indiquant un pied plat, un groupe indiquant un pied creux et un groupe indiquant un pied standard.

11. Dispositif d'évaluation (1) selon la revendication 1, dans lequel le processeur (11) est configuré pour, sur la base de l'au moins une valeur d'indice, faire au moins une des propositions : une chaussure pour le sujet ; une forme à chaussure pour le sujet ; une semelle intérieure de la chaussure pour le sujet ; des soins des pieds pour le sujet ; et de l'entraînement pour le sujet.

12. Dispositif d'évaluation (1) selon la revendication 1, dans lequel
le processeur (11) est configuré pour
calculer, à partir des données tridimensionnelles, au moins deux valeurs d'indice parmi la valeur d'indice liée au plan frontal du pied, la valeur d'indice liée au plan sagittal du pied et la valeur d'indice liée au plan horizontal du pied, et
évaluer un type de pied plat du pied du sujet sur la base des au moins deux valeurs d'indice.

13. Dispositif d'évaluation (1) selon la revendication 12, comprenant en outre un affichage (3) qui montre une image (201) indiquant le niveau de pied plat et une image (204A) indiquant le type de pied plat, dans lequel
l'image (204A) indiquant le type de pied plat comporte des informations sur des caractéristiques desdites au moins deux valeurs d'indice.

14. Dispositif d'évaluation (1) selon la revendication 1, dans lequel
le processeur (11) est configuré pour
spécifier un premier score de valeur d'indice sur la valeur d'indice liée au plan sagittal du pied, et un second score de valeur d'indice sur au moins une valeur d'indice parmi la valeur d'indice liée au plan frontal du pied et la valeur d'indice liée au plan horizontal du pied, et
évaluer le niveau de pied plat en accordant davantage de poids au premier score de valeur d'indice qu'au seconde score de valeur d'indice.

15. Dispositif d'évaluation (1) selon la revendication 1, dans lequel
l'au moins une valeur d'indice comporte une première valeur,
le processeur (11) est configuré pour évaluer le niveau de pied plat sur la base du fait que la première valeur appartient ou non à une première plage prescrite, et
la première plage prescrite est définie sur la base d'une valeur idéale de la première valeur comme une référence.

16. Dispositif d'évaluation (1) selon la revendication 15, dans lequel
la première valeur comporte au moins un parmi :
comme la valeur d'indice liée au plan frontal, un angle formé entre un axe vertical du pied et une ligne médiane du talon du pied ;
comme l'indice lié au plan sagittal, un rapport de hauteur de voûte ; et
comme la valeur d'indice liée au plan horizontal, une zone d'une partie située à un côté médial du pied, d'une ligne droite reliant un point de contour indiquant une largeur de talon du pied et un point de contour d'une partie de milieu du pied, dans une vue en plan le long du plan horizontal du pied.

17. Dispositif d'évaluation (1) selon la revendication 1, dans lequel
l'au moins une valeur d'indice comporte une deuxième valeur,
le processeur (11) est configuré pour évaluer le niveau de pied plat sur la base du fait que la deuxième valeur appartient ou non à une deuxième plage prescrite, et
la deuxième plage prescrite est définie sur la base de, comme une référence, une valeur moyenne des deuxièmes valeurs dans une population prescrite.

18. Dispositif d'évaluation (1) selon la revendication 17, dans lequel
la deuxième valeur comporte au moins un parmi :
la valeur d'indice liée au plan frontal ; et
la valeur d'indice liée au plan horizontal.

19. Dispositif d'évaluation (1) selon la revendication 1, dans lequel
l'au moins une valeur d'indice comporte une troisième valeur,
le processeur (11) est configuré pour évaluer le niveau de pied plat sur la base du fait que la troisième valeur appartient ou non à une troisième plage prédéfinie, et
la troisième plage prescrite est définie sur la base de, comme une référence, une valeur de coupure de la troisième valeur dans une population prescrite.

20. Dispositif d'évaluation (1) selon la revendication 19, dans lequel la troisième valeur comporte au moins une valeur comportée dans la valeur d'indice liée au plan sagittal.

21. Dispositif d'évaluation (1) selon la revendication 1, dans lequel
la mémoire (12) est configurée pour stocker des informations de plage dans lesquelles une plage prescrite correspondant à l'au moins une valeur d'indice est définie en association avec chaque attribut du sujet, et
le processeur (11) est configuré pour
acquérir l'attribut du sujet,
se référer aux informations de plage pour spécifier la plage prescrite correspondant à l'attribut acquis et correspondant à l'au moins une valeur d'indice, et
évaluer le niveau de pied plat sur la base du fait que l'au moins une valeur d'indice appartient ou non à la plage prescrite spécifiée.

22. Procédé d'évaluation d'évaluer une forme d'un pied d'un sujet, le procédé d'évaluation comprenant :
calculer, à partir de données tridimensionnelles de la forme du pied du sujet mesurées par un dispositif de mesure (2), au moins une valeur d'indice parmi une valeur d'indice liée à un plan frontal du pied, une valeur d'indice liée à un plan sagittal du pied et une valeur d'indice liée à un plan horizontal du pied ; et
évaluer un niveau de pied plat du pied du sujet sur la base de l'au moins une valeur d'indice,
**caractérisé en ce que**
la valeur d'indice liée au plan sagittal comporte
une valeur d'indice de voûte liée à une voûte du pied, et
une valeur d'indice de non-voûte différente de la valeur d'indice de voûte, et
au moins la valeur d'indice liée au plan sagittal du pied à partir des données tridimensionnelles est calculée,
dans lequel
la valeur d'indice de non-voûte comporte un angle entre une ligne droite formée par une part de coup-de-pied du pied et une ligne droite formée par une ligne horizontale du pied.
